# EUROPÄISCHE PATENTSCHRIFT

(11) **EP 4 561 484 B1**
(45) Veröffentlichungstag und Bekanntmachung des Hinweises auf die Patenterteilung: **02.09.2026**
(21) Anmeldenummer: 23748487.8
(22) Anmeldetag: 25.07.2023
(51) Int. Cl.: A61B 34/20, A61B 90/50, A61B 90/00, A61B 34/30, G16H 30/40, G06T 7/73, G16H 20/40, G16H 30/20, G16H 40/63, G16H 50/20

(54) **NAVIGATIONSSYSTEM MIT ANNOTATIONSFUNKTION**
NAVIGATION SYSTEM HAVING AN ANNOTATION FUNCTION
SYSTÈME DE NAVIGATION AYANT UNE FONCTION D'ANNOTATION

(30) Priorität: 28.07.2022 DE 102022118990
(43) Veröffentlichungstag der Anmeldung: 04.06.2025
(73) Patentinhaber: B. Braun New Ventures GmbH, 79110 Freiburg im Breisgau (DE)
(72) Erfinder: HEINZE, Peter, 76199 Karlsruhe (DE)
(74) Vertreter: Winter, Brandl - Partnerschaft mbB
(86) Internationale Anmeldenummer: PCT/EP2023/070610
(87) Internationale Veröffentlichungsnummer: WO 2024/023102

(56) Entgegenhaltungen:
- WO-A1-2019/117926
- WO-A1-2019/174953
- WO-A1-2020/023186
- WO-A1-2022/079715
- JP-A- 2022 102 041
- US-A1- 2019 247 130
- US-A1- 2019 262 084
- US-A1- 2021 153 953

## Beschreibung

### Technisches Gebiet

Die vorliegende Offenbarung betrifft ein chirurgisches Navigationssystem mit einer Annotationsfunktion/ einer Markierungsfunktion für eine Eingabe von positionsbezogenen, insbesondere visuellen, medizinischen Annotationen/ Markierungen oder Aufnahmen und/oder für eine Ausgabe von medizinischen Annotationen/ Markierungen bei einem chirurgischen Eingriff bei einem Patienten mit: einer (visuellen) Darstellungsvorrichtung, insbesondere einem OP-Monitor oder einem Display oder einer AR-Brille oder einer VR-Brille, zur visuellen Ausgabe der medizinischen Annotationen; und zumindest einem, vorzugsweise mehrere unabhängig voneinander aktuierbare, Roboter als Positioniersystem, mit (jeweils) einem an einer Roboterbasis beweglich angebundenen, insbesondere mehrgliedrigem, Roboterarm und einem an den Roboterarm endständig angeordneten Roboterkopf. An den Roboterkopf kann ein zu positionierendes Modul, insbesondere ein Endeffektor wie ein Instrument, angebunden werden.

### Hintergrund der vorliegenden Offenbarung

Aus dem Stand der Technik der Druckschrift WO 2020/023186 A1 ist bereits ein Verfahren zur Durchführung eines chirurgischen Eingriffs bekannt. Das Verfahren umfasst das Speichern einer Softwareanwendung in einem mit einem Computer verbundenen Speicher, die, wenn sie von einem Prozessor ausgeführt wird, die Softwareanwendung veranlasst, ein Modell der anatomischen Struktur eines Patienten zu entwickeln, Bilder der Anatomie des Patienten zu verarbeiten, die Bilder der Anatomie des Patienten auf einer mit dem Computer verbundenen Benutzeroberfläche anzuzeigen, kritische Strukturen innerhalb des Patienten über die angezeigten Bilder der Anatomie des Patienten zu legen, eine Stelle innerhalb der Körperhöhle des Patienten zu bestimmen, an der die Bilder der Anatomie des Patienten aufgenommen wurden, und das Modell der anatomischen Struktur des Patienten auf der Benutzerschnittstelle anzuzeigen, wobei das angezeigte Modell die bestimmte Stelle angibt, an der die Bilder der Anatomie des Patienten aufgenommen wurden.

Im Bereich der chirurgischen Navigation existieren aktuell allgemein im Wesentlichen zwei unterschiedliche Gruppen/ Arten/ Typen/ Klassen von chirurgischen Navigationssystemen.

Die erste Gruppe der chirurgischen Navigationssysteme verwendet Aufnahmen/ Bilder wie Computertomografie-Aufnahmen (CT-Aufnahmen), Magnetresonanztomografie-Aufnahmen (MRT-Aufnahmen), Röntgen-Aufnahmen, Ultraschall-Aufnahmen, die präoperativ, also vor dem eigentlichen Eingriff am Patienten erzeugt werden, eine sogenannte imagebased navigation/bildbasierte Navigation. Die präoperativen Aufnahmen müssen mit der Position oder Lage des Patienten während des Eingriffs korreliert/ abgeglichen werden, um eine entsprechende Navigation von Instrumenten oder Implantaten zu ermöglichen. Der Patient wird dabei gegenüber den präoperativen Aufnahmen registriert, um die virtuelle Anatomie des Patienten der präoperativen (3D-)Aufnahmedaten mit der intraoperativen (zeitaktuellen) realen Anatomie zu korrelieren und Instrumente gegenüber den präoperativen Aufnahmedaten anzuzeigen.

Die zweite Gruppe der chirurgischen Navigationssysteme arbeitet ohne Aufnahmen bzw. Bilder (Imageless-Navigation/bildlose Navigation). Diese Navigationssysteme erstellen während des Eingriffs, also intraoperativ, ein Modell des Patienten, in der Regel durch Abtasten anatomischer Landmarken/ Orientierungspunkte mit einer Navigationssonde/ einem Pointer/ einem Navigationszeiger oder durch eine kinematische Analyse mit entsprechenden vereinfachten Achsmodellen.

Die erste Gruppe der Navigationssysteme, die bildbasierten Navigationssysteme, haben den Nachteil einer komplexen und zeitaufwändigen Registrierung, die oftmals nicht die erforderliche Genauigkeit liefert, die für den Eingriff jedoch gefordert wird.

Im Gegensatz dazu erfordern bildlose Navigationssysteme keine präoperative Bildgebung/Aufnahmen und keine intraoperative Registrierung, haben jedoch den Nachteil, dass sie nur ein sehr rudimentäres Modell der Anatomie des Patienten als Referenz verwenden, beispielsweise ein Achsenmodell eines Beins im Falle einer Knieoperation.

Bei einer orthopädischen Navigation gibt es aktuelle Bestrebungen dahingehend, die Vorteile beider Technologien zu kombinieren, das heißt keine präoperative Bildgebung und intraoperative Registrierung mehr zu benötigen, jedoch eine möglichst genaue Aufnahme des Patienten bzw. des Eingriffsbereichs des Patienten als Referenz zu haben, um Instrumente oder Implantate entsprechend auszurichten und für den Patienten schließlich das beste Ergebnis zu erzielen.

Die bildbasierten Navigationsverfahren sind derzeit der Standard in der modernen computergestützten Chirurgie. Ein zentraler Bestandteil der bildgeführten/bildbasierten Navigation ist eine Computertomografie-Visualisierung/ CT-Visualisierung von Zielpositionen, Zieltrajektorien und der Anzeige von CT-Risikoobjekten/ Strukturen in Bezug auf die präoperativ erfassten medizinischen Aufnahmedaten. Diese (Führungs-)Informationen werden aktuell einzig und allein aus den präoperativ erfassten medizinischen Bilddaten abgeleitet, also zeitlich gesehen vor dem Eingriff, und werden während bestimmter Schritte des Eingriffs als primäre Führungsmodalität verwendet.

Üblicherweise wird bei der bildbasierten Navigation ein optisches Trackingsystem (als Messsystem), insbesondere ein aktives oder passives optisches Infrarotsystem, oder ein elektromagnetisches Trackingsystem verwendet, um die präoperativen Aufnahmen bzw. deren Informationen über angebrachte Marker, insbesondere reflektierende Referenzpunkte, an allen notwendigen Objekten auf den Eingriffsbereich/ Operationsort auszurichten. In Folge ist die Visualisierung dieser präoperativen Informationen in Bezug auf die aktuelle intraoperative mikroskopische oder endoskopische (zeitaktuelle) Live-Ansicht des Eingriffsbereichs/ Operationsgebiets des Patienten leider sehr ungenau. Insbesondere kann diese Ungenauigkeit auf einer ungenauen Registrierung basieren. Eine besonders hohe Ungenauigkeit kann dadurch auftreten, dass eine Anatomie des Patienten zwischen einer präoperativen (Bild-)Aufnahme und einer intraoperativen Situation verschoben wird, wie dies beispielsweise bei einem sogenannten Brainshift bei Öffnung eines Schädels des Patienten der Fall ist.

Insbesondere beeinflussen dabei die folgenden Aspekte die Ungenauigkeit bzw. Genauigkeit der Navigation: ein Tracking-Fehler für jeden einzelnen nachverfolgten Starrkörper (rigid body/ fiducial set); ein Fehler der Patientenregistrierung; ein Fehler bei der Aufnahmerekonstruktion; eine Veränderungen der Anatomie in Bezug auf die präoperativen Aufnahmedaten, beispielsweise durch eine Gewebeverschiebung wie einen sogenannten Brainshift; ein Fehler in einer Hand-Auge-Kamera-Kalibrierung; und/oder ein Fehler in einer optischen (Kamera-)Kalibrierung.

Zudem erfordern die optischen Tracking-Technologien ebenfalls eine (kontinuierliche) Sichtlinie zwischen der Trackingkamera einerseits und einem nachzuverfolgenden Instrument andererseits, was wiederum den tatsächlich möglichen Arbeitsbereich des Chirurgen stark einschränkt. Eine ungünstige Position des Chirurgen etwa oder anderer Instrumente oder eines Roboterarms kann die Sicht verdecken bzw. die Sichtlinie unterbrechen, was zu einer unerwünschten Unterbrechung der Nachverfolgung/ des Trackings während des Eingriffs führt und eine Sicherheit des Patienten gefährdet. Auch verlängert sich dadurch die Eingriffsdauer.

### Zusammenfassung der vorliegenden Offenbarung

Es sind daher die Ziele und Aufgabe der vorliegenden Offenbarung, die Nachteile aus dem Stand der Technik zu vermeiden oder zumindest zu mindern und insbesondere ein chirurgisches Navigationssystem mit Annotationsfunktion zur Verfügung zu stellen, welches eine verbesserte Navigation, insbesondere eine höhere Präzision einer Navigation, mit verbesserter, flexibler Annotation bereitstellt. Eine weitere Teilaufgabe kann darin gesehen werden, aus verschiedenen Teilsystemen Informationen zu sammeln und zentral zu hinterlegen und für eine Navigation entsprechend zentral einzubinden, um auf alle Daten geordnet zurückgreifen zu können. Insbesondere liegt eine Teilaufgabe darin, auch ohne präoperative Informationen (Aufnahmen) während des Eingriffs intraoperativ eine Navigation mit hoher Genauigkeit bereitzustellen.

Die Aufgaben der vorliegenden Offenbarung werden hinsichtlich eines gattungsgemäßen chirurgischen Navigationssystems erfindungsgemäß durch die Merkmale des Anspruchs 1 gelöst. Vorteilhafte Weiterbildungen sind Gegenstand der abhängigen Ansprüche.

Ein Kerngedanke der vorliegenden Offenbarung ist also die zeitaktuelle, intraoperative Erfassung (also während des Eingriffs am Patienten) von charakteristischen Punkten oder Objekten in einer Aufnahme, insbesondere 3D-Aufnahme, und eine entsprechende Registrierung (oder Positionserkennung, insbesondere Lagererkennung der Punkte, Objekte oder Bereiche von Gewebestrukturen) und Speicherung mit zumindest den entsprechenden Koordinaten (X, Y und Z-Koordinaten) in einem (einzigen) zentralen, globalen Koordinatensystem. Das globale Koordinatensystem kann gegenüber dem Patienten ausgerichtet sein, etwa als Koordinatensystem des Patienten ausgebildet sein, oder alternativ am Navigationssystem ausgerichtet sein, insbesondere mit einem Nullpunkt des Koordinatensystems (KOS) des Trackingsystems, vorzugsweise einem Nullpunkt in einer Trackingkamera. Entscheidend ist lediglich, dass alle Informationen in ein einziges zentrales Koordinatensystem überführt werden, so dass eine spätere Umrechnung sowie Fehlerquellen entfallen.

In die Bestimmung der (Positions-)Koordinaten des Annotationsobjekts gehen die verschiedenen Positionsdaten ein, die durch eine Kombination von Navigation (globale Positionserfassung), einer Visualisierung durch das Aufnahmesystem (lokale Positionserfassung) und des Roboters bzw. der Robotik (sensorische Positionserfassung über den Roboterarm) einfließen. Mit anderen Worten kann gemäß der vorliegenden Offenbarung eine Verbesserung der Navigation durch eine Kombination aus Visualisierung, Robotik und Navigation erzielt werden.

Mit noch anderen Worten wird ein chirurgisches Navigationssystem mit Annotationsfunktion/ Markierungsfunktion für eine Eingabe von positionsbezogenen, insbesondere visuellen oder auch von Nervensignal- oder Histologie-Informationen an einer bestimmten Stelle der Anatomie, medizinischen Annotationen oder Aufnahmen und/oder für eine Ausgabe von medizinischen Annotationen bei einem chirurgischen Eingriff bei einem Patienten, bereitgestellt, mit: einer Darstellungsvorrichtung, insbesondere einem OP-Monitor, zur visuellen Ausgabe der medizinischen Annotationen; einem Roboter als Positioniersystem, mit einem an einer Roboterbasis beweglich angebundenen, insbesondere mehrgliedrigem, Roboterarm und einem an den Roboterarm endständig angeordneten Roboterkopf, wobei das Navigationssystem ferner aufweist: einen an oder in dem Roboterkopf angeordneten stereoskopischen Aufnahmesystem, insbesondere einer Stereokamera, besonders bevorzugt einem Stereomikroskop, mit zumindest zwei zueinander beabstandeten Aufnahmeeinheiten, um eine (zeitaktuelle) 3D-Aufnahme einer Struktur oder Oberfläche (eines Zielbereichs) gegenüber dem Aufnahmesystem durchzuführen und diese zusammen mit Aufnahmeparametern (des Aufnahmesystems) computerlesbar bereitzustellen; ein Trackingsystem, das dafür angepasst ist, insbesondere mittelbar über die Lage (also der Position und der Orientierung) des Roboterkopfs, die Lage des Aufnahmesystems zu erfassen und nachzuverfolgen und die Lage des Aufnahmesystems computerlesbar bereitzustellen; einem Annotationssystem/ Markierungssystem mit einer Speichereinheit, in welcher eine digitale Annotationsdatenbank/ Markierungsdatenbank mit einem globalen Koordinatensystem gespeichert/hinterlegt ist; und einer Steuereinheit, die dafür angepasst ist, die bereitgestellte 3D-Aufnahme zusammen mit den Aufnahmeparametern und der bereitgestellten Lage zu verarbeiten und basierend auf den bereitgestellten Aufnahmeparametern und der Lage des Aufnahmesystems eine Lage der 3D-Aufnahme in dem globalen Koordinatensystem zu bestimmen und über die Darstellungsvorrichtung in einer Überlagerungsdarstellung zumindest ein Annotationsobjekt der Annotationsdatenbank (welches ebenfalls in dem globalen Koordinatensystem gespeichert und damit zu der Lage der 3D-Aufnahme korreliert ist) in der 3D-Aufnahme zumindest positionskorrekt, insbesondere lagekorrekt, auszugeben und eine Navigation zu verbessern. Es kann also insbesondere zu der (zeitaktuellen intraoperativen) 3D-Aufnahme zu jedem Zeitpunkt und beliebiger Position des Aufnahmesystems, das gespeicherte Annotationsobjekt bzw. die Annotation(en) auf der 3D-Aufnahme positionskorrekt, insbesondere lagekorrekt, überlagert dargestellt werden (sofern sich die Position, insbesondere Lage, der Annotation im Sichtbereich des Aufnahmesystems befindet, also in der 3D-Aufnahme).

Insbesondere kann die Steuereinheit dafür angepasst sein, bei einem (von einem Nutzer eingegebenen) Annotationsbefehl/ Markierungsbefehl, basierend auf den bereitgestellten Aufnahmeparametern und der Lage des Aufnahmesystems eine Lage der 3D-Aufnahme zu bestimmen, und als Annotationsobjekt/ Markierungsobjekt zumindest einen Teilausschnitt oder einen Zielbereich der 3D-Aufnahme zusammen mit den Aufnahmeparametern in der Annotationsdatenbank des Annotationssystems in dem globalen Koordinatensystem in der bestimmen Position, insbesondere Lage, zu speichern, um zu einem späteren Zeitpunkt über die Darstellungsvorrichtung in einer Überlagerungsdarstellung das Annotationsobjekt positionskorrekt, insbesondere lagekorrekt, auszugeben und eine Navigation zu verbessern. Mit diesem Navigationssystem können Annotationen zentral mit besonders hoher Genauigkeit hinsichtlich der Positionsdaten, insbesondere Lagedaten, hinterlegt werden.

Mit noch ganz anderen Worten wird ein neuartiges chirurgisches Navigationssystem bereitgestellt, das ein oder mehrere stereoskopische Aufnahmesysteme/ optische Kamerasysteme aufweist, die an einem Positionierungssystem (insbesondere einem Roboterarm, einem optischen Nachverfolgungssystem, einem elektromagnetischen System) angebracht/ befestigt oder angeordnet sind. Das (primäre) Aufnahmesystem wird intraoperativ (also während des Eingriffs) verwendet, um eine (zeitaktuelle) (3D-)Aufnahme wie etwa eine Live-3D-Videoansicht der Operationsstelle/ Eingriffsstelle auf der Darstellungsvorrichtung anzuzeigen bzw. visuell auszugeben. Bildinterne oder räumliche Annotationen/ Anmerkungen können manuell oder automatisch durch zusätzliche Nachverfolgungssysteme (Messsysteme oder Trackingsysteme) auf dem Live-Videobild des Operationsfeldes/ des Eingriffsbereichs gesetzt werden. Diese bildinternen oder räumlichen Anmerkungen werden in demselben (einzigen) globalen Koordinatensystem erfasst und gespeichert und können daher vom Benutzer bei Bedarf zur chirurgischen Führung visualisiert und wiederhergestellt werden. Dies ist mit hoher Reproduzierbarkeit und höherer Gesamtgenauigkeit möglich, auch wenn das Positionierungssystem (Roboterarm, optisches Trackingsystem, elektromagnetisches System) zwischenzeitlich bewegt und die Kameraparameter, beispielsweise ein Fokuspunkt oder eine Fokusebene, geändert wurden. Der Begriff "Fokuspunkt" definiert hierbei einen Punkt im Raum, welcher in einer Fokusebene des optischen Systems des Aufnahmesystems bzw. der Aufnahmeeinheit liegt und insbesondere auf der optischen Achse.

Insbesondere wird beispielsweise über den Roboter eine roboterkinematikbasierte Lagebestimmung des stereoskopischen Aufnahmesystems in dem Koordinatensystem des Roboters und über das Aufnahmesystem eine Lagebestimmung der 3D-Aufnahme in dem Koordinatensystem des Aufnahmesystems durchgeführt. Diese beiden Lagebestimmungen werden miteinander verbunden, indem etwa, ausgehend von der bestimmten Lage des Aufnahmesystems im Roboterkoordinatensystem, die bestimmte Lage der 3D-Aufnahme in das Roboterkoordinatensystem transformiert wird, um so schließlich, ähnlich einer seriellen Transformation, die Lage der 3D-Aufnhame gegenüber dem Roboter (bzw. dessen Roboterkoordinatensystem) zu bestimmen. Hiernach wird diese Lage der 3D-Aufnahme, etwa durch eine festgelegte oder bestimmbare Transformation zwischen dem Roboterkoordinatensystem zu dem globalen Koordinatensystem (dies kann in einer Ausführungsform das (virtuelle) Koordinatensystem des Patienten/Patientenkoordinatensystem sein), in dem globalen Koordinatensystem bestimmt. Mit anderen Worten können aus unterschiedlichen Modalitäten Daten für eine Annotation gewonnen werden, die in ein einziges globales Koordinatensystem überführt bzw. transformiert werden und dort mit entsprechender Position (mit X, Y, Z Koordinaten im Raum), insbesondere entsprechender Position und Orientierung (also Lage), in dem globalen Koordinatensystem gespeichert werden. Auf diese Weise sind nicht mehr unterschiedliche Annotationen in unterschiedlichen Datenbanken mit unterschiedlichen (lokalen) Koordinatensystemen vorgesehen, sondern die Annotation werden in eine gemeinsame Datenbank mit einem gemeinsamen globalen Koordinatensystem integriert. Dadurch wird gewissermaßen eine Standardisierung oder Normierung vorgenommen und durch die zentral hinterlegten Annotationen ein einheitliches System geschaffen (beispielsweise einer Datenbank mit jeweils normierten Annotationen in Form eines Daten-Arrays mit: Annotations-ID, Annotations-Position, Annotations-Orientierung, Annotations-Daten (etwa ein Bild, eine Vektorgrafik, ein Text oder ähnliches) und optional einer Annotations-Transparenz (0% bis 100%, bei 100% ist Annotation ausgeschaltet/ nicht sichtbar)). Diese Annotationen können dann ausgewählt und insbesondere computerbasiert gerendert werden, dass diese in einer Überlagerungsdarstellung mit einer 3D-Aufnahme des Aufnahmesystems positionskorrekt (also an der richtigen Position gegenüber der 3D-Aufnahme und damit des Patienten), insbesondere lagekorrekt (also an der korrekten Position in der korrekten Orientierung), überlagert eingeblendet werden. Wenn insbesondere der Patient nachverfolgt und registriert wird, so ist es von Vorteil, als globales Koordinatensystem ein Patientenkoordinatensystem zu verwenden, das entsprechend korreliert wird. Auf diese Weise können in einer 3D-Aufnahme unterschiedliche Annotationen der zentralen Datenbank mit einheitlichem globalen Koordinatensystem angezeigt werden, etwa als intransparent oder teiltransparente Überlagerung.

Insbesondere wird vorliegend also ein chirurgisches Navigationssystem beschrieben, das zumindest ein stereoskopisches optisches Aufnahmesystem aufweist, das an einem Positionierungssystem (insbesondere Roboterarm) angebracht oder angeordnet ist, das eine intraoperative 3D-Aufnahme erstellt, um eine Live-Videoansicht (Live-3D-Aufnahme) der Operationsstelle zu zeigen. Weitere Kameras können zusätzlich eingesetzt werden, um weitere räumliche und visuelle Informationen vom Operationsgebiet zu erfassen. Das am Positionierungssystem (insbesondere Roboterarm, optisches Tracking-System, elektromagnetisches Tracking) angebrachte Aufnahmesystem kann vom Nutzer, etwa einem Chirurgen, an bestimmte räumliche Positionen bewegt und insbesondere hinsichtlich der Orientierung ausgerichtet werden, wobei das Aufnahmesystem dem Nutzer eine Live-Sicht auf das Operationsgebiet bereitstellt.

Der Chirurg kann dann entweder eine manuelle Methode verwenden, um verschiedene anatomische oder physiologische Regionen zu identifizieren und zu beschriften, beispielsweise in der ausgegebenen 3D-Aufnahme mit einem Mauszeiger einen Punkt als Annotation setzen, oder es können zusätzliche Messsysteme und Methoden zu Anwendung kommen, um insbesondere entsprechende Regionen automatisch zu annotieren, vorliegend zu beschriften. Dabei wird zumindest eine 3D-Aufnahme, insbesondere ein Live-Videobild (Annotationsreferenzaufnahme), sowie dessen räumliche Position (Koordinaten) über das Positionierungssystem (insbesondere über die aktuelle Roboterarmkonfiguration) und aktuelle Kameraeinstellungen zusammen als Annotationsobjekt in das globale Koordinatensystem überführt und gespeichert.

Im Gegensatz zu bestehenden Wegpunktfunktionalitäten in robotergestützten Mikroskopen oder Annotationen in Mikroskopsystemen speichert das vorliegende Navigationssystem sowohl mehr als auch präzisere Daten und Merkmale für eine genaue und bequeme Visualisierung der Annotationsobjekte in Bezug auf die Anatomie des Patienten. Ferner bietet das Navigationssystem die Möglichkeit, das gesamte Navigationssystem zu bewegen oder die Kameraeinstellungen während des Eingriffs zu ändern und dennoch die Annotationsobjekte wiederherzustellen und zu visualisieren. Durch das offenbarte Navigationssystem können Veränderungen der anatomischen Strukturen sicher erkannt werden, da die Daten intraoperativ und nicht präoperativ erfasst werden.

Der Begriff "Trackingsystem" beschriebt etwa ein technisches System, das eine räumliche Lokalisierung ermöglicht und eine Position und/oder Orientierung erfassen kann.

Der Begriff "Position" meint eine geometrische Position im dreidimensionalen Raum, der insbesondere mittels Koordinaten eines kartesischen Koordinatensystems angegeben wird. Insbesondere kann die Position durch die drei Koordinaten X, Y und Z angegeben werden.

Der Begriff "Orientierung" wiederum gibt eine Ausrichtung (etwa an der Position) im Raum an. Man kann auch sagen, dass durch die Orientierung eine Ausrichtung angegeben wird mit Richtungs-bzw. Drehungsangabe im dreidimensionalen Raum. Insbesondere kann die Orientierung mittels drei Winkeln angegeben werden.

Der Begriff "Lage" umfasst sowohl eine Position als auch eine Orientierung. Insbesondere kann die Lage mittels sechs Koordinaten angegeben werden, drei Positionskoordinaten X, Y und Z sowie drei Winkelkoordinaten für die Orientierung.

Der Begriff "positionskorrekt" definiert, dass die Annotation bzw. 3D-Aufnahme an der tatsächlichen, korrekten Position in der Überlagerungsdarstellung wiedergegeben wird, ähnlich eines CAD-Systems.

Der Begriff "lagekorrekt" definiert die Kombination aus positionskorrekt und orientierungskorrekt, bei der die Aufnahme sowohl an der richtigen Position als auch in der richtigen Orientierung in der Überlagerungsdarstellung angezeigt wird. Bei einer parallelen, kohärenten Ausrichtung der Aufnahmerichtungen, wird die überlagerte Aufnahme mit gleichbleibenden Seitenverhältnissen wie in der tatsächlichen Aufnahme dargestellt. Wird nun etwa die Aufnahmerichtung angewinkelt, so wird eine Erstreckung bzw. eine Dimension der Aufnahme der zugehörigen Richtung entsprechend dem Winkel gestaucht, bis bei einem 90° Winkel zwischen den beiden Aufnahmerichtungen nur noch eine eindimensionale Linie in der Überlagerungsdarstellung erzeugt wird.

Der Begriff "Zielbereich" beschreibt hier einen Teil eines Eingriffsbereichs, der von Interesse ist und durch das Aufnahmesystem anvisiert werden soll. Insbesondere kann der Zielbereich ein einzelner Zielpunkt im Raum sein (mit X, Y, Z Koordinate) und kann auch als solche Koordinate angegeben werden. Der Zielbereich kann aber auch ein Ausschnitt aus einer zweidimensionalen Ebene, etwa ein kleiner Ausschnitt einer Gewebeoberfläche, sein. Auch kann ein Zielbereich als ein kleines würfelförmiges oder kugelförmiges Volumen angegeben werden.

Im Gegensatz zu einer bildbasierten Navigation hat das vorgeschlagene Navigationssystem nur noch folgende, negative Einflussfaktoren auf die Genauigkeit: Hand-Augen-Kalibrierung zwischen Kamera und Roboter-Basiskoordinatensystem; und ein kalibriertes Kamerasystem, die zu beachten sind und entsprechend genau gehalten werden können.

Gemäß der Offenbarung werden als Aufnahmeparameter eine Roboterarmkonfiguration des Roboters und eine Kameraeinstellung des Aufnahmesystems in der Annotationsdatenbank gespeichert, um den Roboter zu einem späteren Zeitpunkt des Eingriffs in genau diese Position und Orientierung mit entsprechender Kameraeinstellung zu bewegen/ zu verfahren und in der Überlagerungsdarstellung die zeitaktuelle 3D-Aufnahme sowie die zuvor gespeicherte 3D-Aufnahme als kohärente Referenz auszugeben, etwa als Nebeneinanderdarstellung oder als transparenter Überlagerungsdarstellung, um eine zeitliche Veränderung zu visualisieren. Insbesondere muss für eine sogenannte Intra-Image-Annotation, also einer Annotation innerhalb des Bildes, die Kamera der Aufnahmeeinheit kalibriert sein, und diese Methode erfordert für die wiederholte Visualisierung, dass das Aufnahmesystem auf den gleichen Zoom und den gleichen Brennpunkt eingestellt ist, wie während des Annotationsvorgangs (kohärente Ansicht).

Gemäß der Offenbarung werden zusätzlich oder alternativ als Aufnahmeparameter ein Zoom(-faktor) und ein Brennpunkt als Kameraeinstellungen der Stereokamera erfasst gespeichert, so dass über die durch das Trackingsystem nachverfolgte Lage des Aufnahmesystems und über den Zoom sowie den Brennpunkt zumindest die Position eines Teilbereichs der 3D-Aufnahme präzise bestimmbar ist und/oder auch für eine Visualisierung wieder herstellbar ist.

Ferner kann vorzugsweise die Steuereinheit dafür angepasst sein, bei einem Ausgabebefehl zu einem in der Annotationsdatenbank gespeicherten Annotationsobjekt, das Aufnahmesystem in die zu dem Annotationsobjekt gespeicherte Lage zu bewegen/ zu verfahren und in der Überlagerungsdarstellung die zeitaktuelle 3D-Aufnahme und das gespeicherte Annotationsobjekt, insbesondere den Teilausschnitt der gespeicherten 3D-Aufnahme, über die Darstellungsvorrichtung ausgeben.

Insbesondere kann die Annotation, insbesondere die Intra-Image-Annotation, auch durch Computer-Vision-Algorithmen unterstützt werden, indem entsprechende Regionen während der Änderung der Zoomstufe und des Brennpunkts/der Brennweite verfolgt werden.

Gemäß einer weiteren bevorzugten Ausführungsform können in der Speichereinheit ferner präoperative 3D-Aufnahmedaten, insbesondere CT-Aufnahmedaten und/oder MRT-Aufnahmedaten und/oder segmentierte Röntgenbilder, gespeichert sein, das chirurgische Navigationssystem dafür angepasst sein, den Patienten gegenüber den präoperativen 3D-Aufnahmedaten zu registrieren, und die Steuereinheit dafür angepasst sein, in der Überlagerungsdarstellung zu der 3D-Aufnahme die präoperativen 3D-Aufnahmedaten für eine weitere Unterstützung mit einzublenden. Mit anderen Worten können also medizinische präoperative Aufnahmedaten/ Bildgebungsdaten (3D-CT-Daten /3D-MRT-Daten) als unterstützende Information zusätzlich mit eingebunden werden, die jedoch nicht als primäre Führungsinformation angezeigt werden, sondern nur als sekundäre Führungsinformation. Die primären Führungsinformationen bilden die intraoperativen Aufnahmen bzw. die zugehörigen Annotationen.

Gemäß einer weiteren Ausführungsform kann die Steuereinheit dafür angepasst sein, bei einer Abweichung des in der Annotationsdatenbank gespeicherten Teilausschnitts der 3D-Aufnahme gegenüber der zeitaktuellen 3D-Aufnahme, insbesondere mittels Bildanalyseverfahren, über eine vorgegebene Toleranz hinaus, eine Warnmeldung in der Überlagerungsdarstellung mit einzublenden.

Insbesondere kann die Steuereinheit dafür angepasst sein, bei einer Abweichung der in der Annotationsdatenbank gespeicherten 3D-Aufnahme gegenüber der zeitaktuellen 3D-Aufnahme, insbesondere mittels Bildanalyseverfahren, über eine vorgegebene Toleranz hinaus, zu bestimmen, dass eine Verschiebung des gesamten Patienten vorliegt, wenn die gespeicherte 3D-Aufnahme oder zumindest der Teilausschnitt der 3D-Aufnahme, insgesamt gegenüber der zeitaktuellen 3D-Aufnahme verschoben ist, und die Steuereinheit zusätzlich vorzugsweise dafür angepasst sein, das Navigationssystem neu gegenüber dem Patienten zu registrieren, insbesondere die Registrierung um genau die ermittelte Verschiebung zu korrigieren.

Ferner kann insbesondere kann die Steuereinheit dafür angepasst sein, bei einer Abweichung der in der Annotationsdatenbank gespeicherten 3D-Aufnahme gegenüber der zeitaktuellen 3D-Aufnahme, insbesondere mittels Bildanalyseverfahren, über eine vorgegebene Toleranz hinaus, zu bestimmen, dass eine Verschiebung nur eines Gewebes des Patienten vorliegt, wenn die gespeicherte 3D-Aufnahme oder zumindest der Teilausschnitt der 3D-Aufnahme, gegenüber der zeitaktuellen 3D-Aufnahme verzerrt, etwa gestaucht, ist und zusätzlich vorzugsweise eine Registrierung gegenüber dem relevanten Gewebe vorzunehmen.

Insbesondere kann die Steuereinheit dafür angepasst sein, ein Instrument in der zeitaktuellen 3D-Aufnahme zu erfassen und in der Überlagerungsdarstellung in eine vordere Ebene zu setzen/ einzublenden, während das zumindest eine Annotationsobjekt in einer Zwischenebene, zwischen der Live-Aufnahme und dem Instrument eingeblendet wird, also in einer zu dem Instrument relativ gesehen hinteren Ebene dargestellt wird, so dass in der Überlagerungsdarstellung die Annotationsobjekte nicht mit dem Instrument interferieren bzw. das Instrument nicht durch die Annotationsobjekte überdeckt wird. Ferner kann insbesondere die Steuereinheit dafür angepasst sein, ein Instrument in der zeitaktuellen 3D-Aufnahme zu erfassen und in der Überlagerungsdarstellung in eine hintere Ebene zu setzen/ einzublenden, während das zumindest eine Annotationsobjekt in einer vorderen Ebene, also in einer zu dem Instrument relativ gesehen vorderen Ebene dargestellt wird. Damit können also Annotationen vor oder hinter dem Instrument liegen. Wenn sie vor dem Instrument liegen, so überdecken diese das Instrument intransparent oder halbtransparent (beispielsweise 70% Deckungskraft), wenn die Annotationsobjekte hinter dem Instrument liegen, so überdeckt das Instrument die Annotation intransparent oder halbtransparent (beispielsweise 70% Deckungskraft).

Gemäß einer weiteren Ausführungsform kann das Aufnahmesystem eine zeitaktuelle/ Live 3D-Aufnahme intraoperativ bereitstellen, welche einerseits über die Darstellungsvorrichtung ausgegeben wird und andererseits bei einem Annotationsbefehl in der Annotationsdatenbank gespeichert wird.

Insbesondere kann in der Annotationsdatenbank zu jedem Annotationsobjekt ein Parameter einer Transparenz zwischen 0 und 1 (also ein kontinuierlicher Wert zwischen 0% und 100%) und damit zwischen einer Ausblendung und einer Einblendung hinterlegt sein, und die Steuereinheit dafür angepasst sein, zusammen mit der zeitaktuellen 3D-Aufnahme alle Annotationsobjekte entsprechend ihrer Transparenz in der Überlagerungsdarstellung zumindest positionskorrekt, insbesondere lagekorrekt, einzublenden.

Gemäß einer Ausführungsform kann die Steuereinheit dafür angepasst sein, einen Schwellenwert zur Anzeige von Umrissen/Konturen zu verändern, insbesondere auf Basis einer manuellen Eingabe, um einen Bereich für den Nutzer bedarfsgerecht zu markieren und Grenzen eines Bereichs entsprechend des eingestellten Grenzwerts zu verschieben.

Insbesondere kann der Kopf des Patienten in Bezug auf die Roboterbasis des Roboters bzw. das Robotersystem fixiert sein. Dies ist insbesondere bei bildbasierten Navigationssystemen üblich. Alternativ kann der Patient mit demselben Positionierungssystem, insbesondere Trackingsystem, verfolgt werden.

Gemäß einer weiteren Ausführungsform kann das Navigationssystem, insbesondere das Aufnahmesystem, dafür angepasst sein, eine Kamerakalibrierung durchzuführen, um Informationen, insbesondere einer räumlichen Pyramide, der Steuereinheit bereitzustellen, wie sich die gesammelten Annotationsobjekte in Bezug auf eine Änderung des Zooms/(der Zoomstufe) oder des Brennpunkts/der Brennweite bewegen, so dass die Steuereinheit dafür angepasst ist, die Kameraeinstellungen während eines Verfahrens zu ändern. Mit anderen Worten erfordert das Navigationssystem ein Positionierungssystem (insbesondere Roboterarm, optisches Tracking-System, elektromagnetisches Tracking) mit einer hohen Wiederholbarkeit der räumlichen Positionierung sowie ein (primäres) Kamerasystem mit einer hohen Wiederholbarkeit von Zoom und Brennpunkt/Brennweite, weswegen es von Vorteil ist, wenn auch das Kamerasystem kalibriert wird.

Vorzugsweise können als assoziierte Daten des Annotationsobjekts eine inkrementelle Nummer/ eine Marker-ID, insbesondere eines nachverfolgten Zeigegeräts (Navigationszeiger/Pointer) und/oder eine Intensitätsinformation einer Fluoreszenzaufnahme und/oder eine konvexe Hülle von Trajektorien und/oder ein sphärisches Abstandsfeld in der Annotationsdatenbank gespeichert werden. Mit anderen Worten können insbesondere als assoziierte Daten von Annotationsobjekten folgende Daten hinterlegt werden: Inkrementelle Nummer/Marken-ID z.B. von einem Zeigegerät / Computer Vision Tracked Instrument; Intensitätsinformationen, beispielsweise von einer Fluoreszenzaufnahme; Konvexe Hüllen, z. B. von Trajektorien; ein Sphärisches Abstandsfeld, etwa aus einem Neuromonitoring. Insbesondere können Annotationen im speziellen sein:
eine physiologische Informationen, insbesondere ein Nervensignal und/oder eine Histologie;
geometrische Objekte, insbesondere ein Punkt, ein Kreis, eine Linie, eine Trajektorie und/oder eine Kontur;
und/oder sekundäre Bild-Informationen, insbesondere eine Fluoreszenz. Durch das Navigationssystem der vorliegenden Offenbarung können die unterschiedlichen Anzeigemodalitäten dort in der (zeitaktuellen) 3D-Aufnahme dargestellt werden, wo diese tatsächlich sind, also positionskorrekt, insbesondere lagekorrekt.

Vorzugsweise kann als manuelle Beschriftungsmethode folgende Methode herangezogen werden: Brennpunkt des Aufnahmesystems, insbesondere des Mikroskops (1D-Objekt mit X-Y-Z Koordinaten im globalen Koordinatensystem, dieser Punkt im Raum kann entsprechend beschriftet werden); ein Cursor/ ein Mauszeiger im Livebild der Darstellungsvorrichtung (mit X-Y Koordinaten sowie einem Offset zum Brennpunkt in der Bildebene als Z-Koordinate); ein navigierter Punkt oder Instrument wie ein Navigationszeiger/ Pointer; Computer-Vision-Methoden zur Erkennung von Kanten oder Gewebemustern; ein Ursprung eines Blutes und Fließrichtung des Blutes über die Zeit (ML erkannt), um die Blutungsquelle zu erfassen.

Insbesondere kann auch eine automatische Beschriftungsmethode als Annotationsmethode Verwendung finden, insbesondere: ein Neuromonitoring und/oder eine Fluoreszenzbildgebung (etwa DUV400, DFS560, DIR800) und/oder andere optische Messverfahren wie OCT oder ähnliche.

Insbesondere kann das Navigationssystem als Annotationsobjekt eine (zeitlich begrenzte) 3D-Videoaufnahme, insbesondere mit einer Länge von im Wesentlichen fünf Sekunden hinterlegen, so dass ein Bewegtbild/ ein Video als Annotationsobjekt gespeichert ist. Mit anderen Worten kann also insbesondere ein 4D-Annotationsobjekt (also 3D mit einer zeitveränderlichen Komponente) gespeichert und entsprechend zu einem weiteren Zeitpunkt erneut wiedergegeben werden. Insbesondere kann diese Videoaufnahme von einem ICG stammen.

Vorzugsweise kann das chirurgische Navigationssystem ferner eine Überblickskamera aufweisen, welche derart ausgerichtet angepasst ist, ein Umfeld des Eingriffsbereichs zu erfassen und computerlesbar bereitzustellen. Mit anderen Worten können zusätzliche Kamerasysteme eine automatische Erzeugung von Annotationsobjekten unterstützen.

Vorzugsweise kann das Navigationssystem dafür angepasst sein, eine Intra-Image/Intra-Wegpunkt-Annotation durchzuführen. Bei der sogenannten Intra-Image/Intra-Wegpunkt-Annotation bewegt der Benutzer das Aufnahmesystem an die gewünschte Position und stellt die Kamera auf die gewünschten Parameter in Bezug auf den Zoom und den Brennpunkt ein (Zoomstufe und Brennpunkt/-weite). Nun ist es möglich, manuell oder automatisch Annotationsobjekte innerhalb der 2D-Aufnahme zu identifizieren. Diese Kategorie erfordert ein Robotersystem mit hoher Reproduzierbarkeit und ein primäres Aufnahmesystem/ Kamerasystem mit hoher Wiederholbarkeit von Zoom und Brennpunkt. Insbesondere setzt der Brennpunkt voraus, dass sich der Roboterkopf, insbesondere ein Roboter-Endeffektor, auch in einer angemessenen Lage zur ursprünglichen Lage bewegt (ein Wegpunkt mit gespeicherten Kameraparametern).

Insbesondere kann eine spezielle Kamerakalibrierung Informationen (räumliche Pyramide) darüber liefern, wie sich die gesammelten Annotationsobjekte in Bezug auf eine Änderung der Zoomstufe oder des Brennpunkts/der Brennweite bewegen. Dies ermöglicht es dem Benutzer, die Kameraeinstellungen während des Verfahrens zu ändern. Herkömmliche Annotationssysteme lassen diese Flexibilität während des Verfahrens nicht zu.

Vorzugsweise kann das Navigationssystem dafür angepasst sein, eine räumliche Annotation durchzuführen. Bei der räumlichen Annotation bewegt der Benutzer das Aufnahmesystem (das stereoskopische Aufnahmesystem/ Kamerasystem) an die gewünschte Position und stellt die Kamera auf die gewünschten Parameter in Bezug auf Zoomstufe und Brennpunkt/-weite ein. Nun ist es möglich, manuell oder automatisch Annotationsobjekte innerhalb der 2D-Aufnahme/ des 2D Bildes zu identifizieren. Über vorzugsweise eine spezielle Kamerakalibrierung (stereoskopisch) können die räumlichen 3D Positionen der Beschriftungsobjekte im Koordinatensystem des Positionierungssystems abgeleitet werden. Diese Kategorie der räumlichen Annotation erfordert ein Robotersystem mit hoher Reproduzierbarkeit und ein Aufnahmesystem mit hoher Wiederholbarkeit von Zoom und Brennpunkt/-weite. Der Brennpunkt setzt voraus, dass sich der Roboterkopf, insbesondere der Roboter-Endeffektor auch in einer Pose oder Lage bewegt, die der ursprünglichen Pose nahe kommt (also eine ähnliche Position und Orientierung innerhalb einer Toleranz; Wegpunkt mit gespeicherten Kameraparametern). Darüber hinaus ist insbesondere eine spezielle Kamerakalibrierung für die 3D-Rekonstruktion erforderlich sowie eine Hand-Augen-Kalibrierung, um die Daten der 3D-Rekonstruktion in das Koordinatensystem des Positionierungssystems zu übertragen. Dies ermöglicht es dem Benutzer, die Kamera zu bewegen und die Kameraeinstellungen frei zu verändern. Die Visualisierung der Annotationsobjekte kann unter Berücksichtigung der angepassten Posen und Kameraeinstellungen visualisiert werden. Auch diese Methode kann vorzugsweise auch durch Computer-Vision-Algorithmen unterstützt werden, indem entsprechende Regionen bei der Änderung von Zoom und Brennpunkt/weite sowie bei Roboterbewegungen nachverfolgt werden.

Insbesondere können folgende Benutzerinteraktionen durch das Navigationssystem realisiert werden bzw. das Navigationssystem dafür angepasst sein, die folgenden Funktionen durchzuführen: Ein- und Ausschalten der Anmerkungsobjekte (etwa durch entsprechende Einträge in der Annotationsdatenbank), ergänzt um die Live-Ansicht; Verschieben eines Schwellenwerts zur Anzeige von Umrissen/Konturen (etwa Tumorgrenzen) aus Punktwolken regelmäßiger Proben mit entsprechender Intensität (z. B. eine Fluoreszenzbild) (Benutzergeführte Segmentierung von Strukturen durch einfaches Einzeichnen (basierend auf sogenannten max-cut-min-flow).

Insbesondere kann eine freie Bewegung des Aufnahmesystems (Kamerakopfes) durch eine Erweiterung der räumlichen Annotationsobjekte erfolgen, was insbesondere eine Kalibrierung erfordert. Das Annotationsobjekt wird in Bezug auf den fixierten Operationsort erweitert.

Vorzugsweise kann in der Überlagerungsdarstellung auch zumindest eine Richtung zu gespeicherten Annotationen angezeigt bzw. eingeblendet werden, insbesondere während einer (automatischen oder manuellen) Bewegung des Roboterarms.

Vorzugsweise kann als eine Vermessung (in mm) von Annotationsobjekten zur Dokumentation erfolgen und als zu dem Annotationsobjekt assoziierte Daten in der Annotationsdatenbank gespeichert werden.

Weiter vorzugsweise kann ein Fokus und Zoom für räumliche Annotationsobjekte gespeichert und/oder entsprechend gesetzt werden, insbesondere auf eine Kontur eines Fluoreszenzbilds.

Insbesondere kann als Annotationsobjekt eine (1D oder 2D) Heatmap/ Temperaturlandkarte ergänzt werden, um Ziel- oder Risikostrukturen besser darzustellen.

Ein wichtiger Teil des Navigationssystems ist die Visualisierung der erfassten und verarbeiteten Annotationen. Die Visualisierung der Annotationsobjekte kann vorzugsweise so angepasst sein, dass die Überlagerung der Objekte nicht mit eingesetzten Instrumenten interferiert (etwa eine Maskierung - ähnlich einer Tiefen-Unschärfe eines Hintergrunds). Dadurch wird die Visualisierung für den Chirurgen verbessert und der Eindruck verstärkt, dass das Anmerkungsobjekt mit dem Operationsgebiet des Patienten verbunden ist und nicht nur ein einfaches Overlay darstellt. Beispielsweise kann in einer Ausführungsform die Steuereinheit dafür angepasst sein, das Annotationsobjekt/ Anmerkungsobjekt so unscharf darzustellen, dass das chirurgische Instrument darüber zu liegen scheint (maskiert). Die Anmerkungen können vorzugsweise auch durch verschiedene Brennpunktbilder (1-2 Aufnahmeebenen nach oben oder unten) mit einem gewissen Grad an Transparenz durchscheinen.

Das Navigationssystem kann insbesondere dafür angepasst sein, erfasste räumliche Anmerkungen ungültig zu markieren, wenn die zugrundeliegende Anatomie durch einen chirurgischen Eingriff verändert worden ist. Dies kann durch den Vergleich des Referenzbildes der Annotation mit der aktuellen Live-Ansicht der Kamera erreicht werden.

Auch kann vorzugsweise das Navigationssystem, insbesondere die Steuereinheit, dafür angepasst sein, die Visualisierung des räumlichen Annotationsobjekts auszublenden, wenn der Visualisierungsfehler nach einer Bewegung des Mikroskopkopfs zunimmt.

Vorzugsweise kann das Navigationssystem auch dafür angepasst sein, eine Biopsie bzw. Biopsieinformationen als Annotationen zu hinterlegen. Insbesondere ist eine automatische Generierung von chirurgischen Dokumentationen leicht möglich, wenn Annotations-Punkte auch diagnostische Informationen liefern, insbesondere aus Biopsien.

Insbesondere können räumliche Annotationsobjekte dazu herangezogen werden, präoperative CT-/MRT-Daten zu registrieren, um während der Operation eine zusätzliche Unterstützung zu erhalten.

### Kurzbeschreibung der Figuren

Die vorliegende Offenbarung wird nachfolgend anhand bevorzugter Ausführungsformen mit Hilfe von Figuren näher erläutert. Es zeigen:
Fig. 1 eine schematische Darstellung eines Navigationssystems nach Stand der Technik, in welche unterschiedliche Fehlerquellen angezeigt sind,
Fig. 2 eine perspektivische Ansicht eines chirurgischen Navigationssystems gemäß einer bevorzugten Ausführungsform der vorliegenden Offenbarung, welche eine Annotation in einer kohärenten Ansicht darstellen kann
Fig. 3 eine weitere perspektivische Ansicht eines chirurgischen Navigationssystems gemäß einer weiteren bevorzugten Ausführungsform, welche ein Annotationsobjekt mit Tiefeninformation erfasst,
Fig. 4 eine perspektivische Ansicht des Navigationssystems aus Fig. 3, welches in einer zweiten Lage die angepasste Darstellung der Annotation erfasst oder ausgibt,
Fig. 5 eine weitere perspektivische Ansicht des Navigationssystems aus Fign 3 und 4, in welcher eine Annotation aus einem Erfassungsbereich herausbewegt wird,
Fig. 6 eine schematische Darstellung einer beispielhaften 3D-Aufnahme mit kreisförmigen und punktförmigen Annotationen,
Fig. 7 eine schematische Darstellung einer beispielhaften 3D-Aufnahme mit einer Kontur-Annotation,
Fig. 8 eine schematische Darstellung einer beispielhaften 3D-Aufnahme mit einer Trajektorie-Annotation,
Fig. 9 eine schematische Darstellung einer beispielhaften 3D-Aufnahme mit einer Fluoreszenz-Annotation,
Fig. 10 eine weitere schematische Darstellung einer beispielhaften 3D-Aufnahme mit einer Fluoreszenz-Annotation,
Fig. 11 eine schematische Darstellung einer beispielhaften 3D-Aufnahme mit einem nachverfolgten Instrument und zugehörigen Distanzfeldern als Annotationen,
Fign. 12 und 13 eine schematische Darstellung einer beispielhaften 3D-Aufnahme mit Bewegungsvektoren einer vaskulären Fluoreszenz (ICG) als gespeicherte Annotationen,
Fign. 14 und 15 eine schematische Darstellung einer beispielhaften 3D-Aufnahme mit einer Biopsie als Annotation,
Fig. 16 eine schematische Darstellung einer beispielhaften 3D-Aufnahme mit unterschiedlich eingestellten Schwellenwerten für eine Erfassung einer Grenze als Annotation,
Fig. 17 eine schematische Darstellung einer beispielhaften 3D-Aufnahme mit einer hinterlegten Abmessung in mm als Annotation,
Fig. 18 eine schematische Darstellung einer beispielhaften 3D-Aufnahme mit einer augmentierten Wärmekarte als Annotation,
Fig. 19 eine schematische Darstellung einer beispielhaften 3D-Aufnahme mit einer wiedergabefähigen 4D-Annotation (Video),
Fig. 20 eine schematische Darstellung einer beispielhaften 3D-Aufnahme mit einem nachverfolgten Instrument und einer in den Hintergrund verlegten Annotation, so dass das Instrument im Vordergrund erscheint.

Die Figuren sind schematischer Natur und sollen nur dem Verständnis der Erfindung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

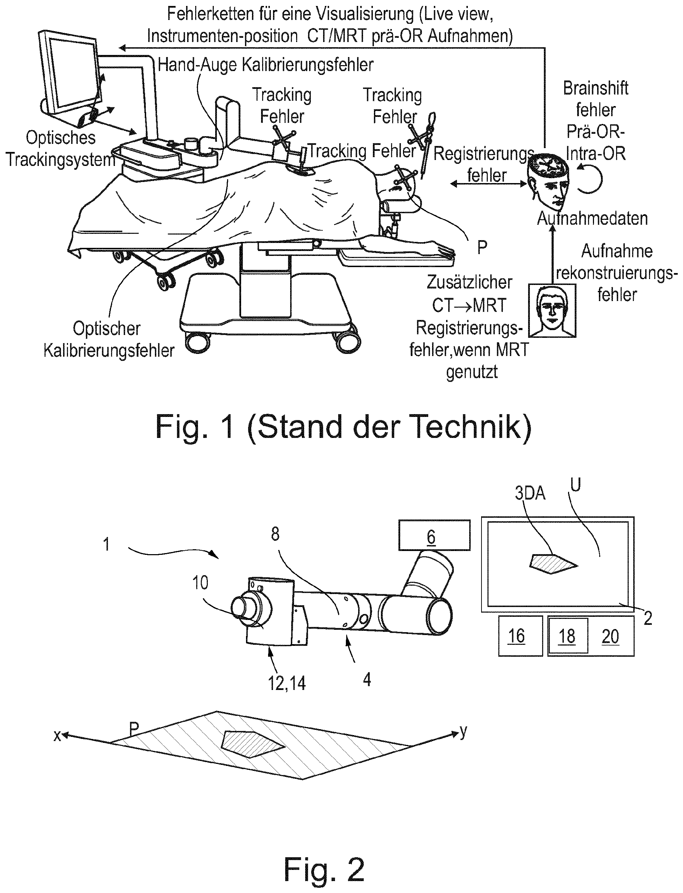

Fig. 1 zeigt in einer perspektivischen schematischen Ansicht ein chirurgisches Navigationssystem gemäß dem Stand der Technik. Hier treten eine Vielzahl von Fehlerquellen auf, welche eine Präzision negativ beeinflussen. Instrumente werden über angebrachte Starrkörper (Infrarotmarker) durch die Trackingkamera nachverfolgt, wobei für jeden einzelnen Starrkörper Tracking-Fehler mit gewisser Fehlertoleranz auftreten. Auch wird ein Patient über einen Starrkörper nachverfolgt mit einhergehendem Tracking-Fehler. Ferner kann es zu einem Fehler bei der Aufnahmerekonstruktion als auch einer Veränderung der Anatomie in Bezug auf die präoperativen Aufnahmedaten kommen, beispielsweise durch eine Gewebeverschiebung wie einen sogenannten Brainshift. Eine weitere Fehlerquelle kann in einer Hand-Auge-Kamera-Kalibrierung liegen oder einer optischen Kalibrierung. Diese Fehler als auch Fehlerketten gilt es vermeiden oder zumindest zu minimieren, um eine noch bessere und präzisere Navigation zu ermöglichen.

Fig. 2 zeigt in einer perspektivischen Ansicht ein chirurgisches Navigationssystem 1 (nachstehend nur Navigationssystem genannt) mit einer Annotationsfunktion. Diese Annotationsfunktion dient dazu, positionsbezogene visuelle medizinische Annotationen und Aufnahmen intraoperativ mit einzubinden und für eine Führung und einen chirurgischen Eingriff an einem Patienten P (hier angedeutet) zu verwenden.

Um die eingegebenen Annotationen visuell darzustellen zu können weist das Navigationssystem 1 eine Darstellungsvorrichtung 2 in Form eines OP-Monitors auf, um die medizinischen Annotationen für ein medizinisches Fachpersonal, insbesondere einen Chirurgen visuell auszugeben.

Das Navigationssystem 1 weist ferner einen Roboter 4 als Positioniersystem auf. Konkret hat der Roboter eine ortsfeste Roboterbasis 6 und einen an die Roboterbasis 6 beweglich angebundenen mehrgliedrigen Roboterarm 8, der aktiv gesteuert und bewegt/ verfahren werden kann. An einem Endabschnitt des Roboterarms 8 ist ein endständiger Roboterkopf 10 beweglich verbunden, der sich zumindest gegenüber einer Längsachse des letzten Segments/Glieds des Roboterarms 8 verdrehen lässt.

In dem Roboterkopf 10 ist ein stereoskopisches Aufnahmesystem 12, insbesondere eine Stereokamera, vorliegend ein Stereomikroskop, mit zumindest zwei zueinander beabstandeten Aufnahmeeinheiten 14, hier Aufnahmekameras, aufgenommen, das über zwei optische Systeme mit jeweils einem nachgeschalteten Sensor eine 3D-Aufnahme 3DA einer Struktur oder Oberfläche aufnimmt. Diese 3D-Aufnahme 3DA wird zusammen mit Aufnahmeparametern, hier insbesondere Kameraparametern, computerlesbar bereitgestellt.

Das Navigationssystem 1 weist zur Nachverfolgung des Roboterkopfs 10 ein Trackingsystem 16 auf, das in der vorliegenden Ausführungsform als mechanischsensorisches Trackingsystem 16 ausgestaltet ist und über die Roboterarmkonfiguration eine Lage des Roboterkopfs 10 gegenüber der Roboterbasis 6 bzw. gegenüber einem Koordinatensystem des Roboters 4 erfasst und mittelbar, über die Lage des Roboterkopfs 10, die Lage des Aufnahmesystems 12 zu erfassen und räumlich nachzuverfolgen. Diese erfasste Lage des Aufnahmesystems 12 wird computerlesbar bereitgestellt.

Für eine Datenverwaltung von Annotationen weist das Navigationssystem 1 ein zentrales internes Annotationssystem 18 mit einer Speichereinheit (hier nicht dargestellt) auf, in welcher eine digitale Annotationsdatenbank mit einem einzigen globalen Koordinatensystem gespeichert ist, wobei vorliegend das globale Koordinatensystem das Koordinatensystem des Roboters 4 ist. In dieser Annotationsdatenbank kann als Listeneintrag (ähnlich einer SQL-Datenbank, ein neues Annotationsobjekt angelegt werden und es können assoziierte Daten zu dem Annotationsobjekt gespeichert werden.

Eine speziell angepasste Steuereinheit 20 (Annotationssystem 18 ist ein Teil/eine Subeinheit der Steuereinheit 20) ist konfiguriert, die bereitgestellte 3D-Aufnahme 3DA zusammen mit den Aufnahmeparametern und der bereitgestellten Lage des Aufnahmesystems zu verarbeiten. Ist ein Annotationsobjekt in der Annotationsdatenbank hinterlegt, so kann die Steuereinheit 20 basierend auf den bereitgestellten Aufnahmeparametern und der Lage des Aufnahmesystems zunächst eine Lage der 3D-Aufnahme 3DA im Raum zu bestimmen, und zwar eine Lage gegenüber dem zentralen globalen Koordinatensystem. Dieses hinterlegte Annotationsobjekt bzw. die Daten des Annotationsobjekts wird dann, wenn es in den Sichtbereich der 3D-Aufnahme fällt, in einer Überlagerungsdarstellung positionskorrekt, insbesondere lagekorrekt ausgegeben. Beispielsweise kann als Annotationsobjekt ein Bild, ein Text oder andere medizinische Daten für einen Eingriff hinterlegt sein und das Bild kann in der 3D-Aufnahme lagekorrekt oder der Text kann in der 3D-Aufnahme positionskorrekt eingeblendet werden.

Ferner ist die Steuereinheit 20 speziell angepasst, die bereitgestellte 3D-Aufnahme 3DA zusammen mit den Aufnahmeparametern und der bereitgestellten Lage des Aufnahmesystems zu verarbeiten und bei einem Annotationsbefehl, etwa über das Touchdisplay des OP-Monitors als Darstellungsvorrichtung oder automatisch basierend auf einem internen Annotationsprogramm, basierend auf den bereitgestellten Aufnahmeparametern und der Lage des Aufnahmesystems zunächst eine Lage der 3D-Aufnahme im Raum zu bestimmen, und zwar eine Lage gegenüber dem zentralen globalen Koordinatensystem. Mit anderen Worten wird ausgehend von der Roboterbasis 6, über den Roboterarm 8, über den Roboterkopf 10, über das Aufnahmesystem 12 und den optischen Parametern schließlich die Lage der 3D-Aufnahme bestimmt. Diese Lage wird in das zentrale globale Koordinatensystem übertragen.

In der Annotationsdatenbank des Annotationssystems wird dann als Annotationsobjekt zumindest ein Teilausschnitt der 3D-Aufnahme, etwa von einem zu interessierenden Bereich, beispielsweise einem Tumor, zusammen mit den Aufnahmeparametern in dem globalen Koordinatensystem in der bestimmen Position hier sogar die bestimmte Lage, gespeichert. Insbesondere wenn das Navigationssystem 1 nicht nur einen Roboter 4, sondern mehrere Roboter 4 mit Roboterarmen aufweist, können wichtige Daten für den Eingriff zentral gespeichert werden, wobei ein (einziges) globales Koordinatensystem alle Annotationen zentral aufnimmt. Man kann sich dieses globale Koordinatensystem wie einen dreidimensionalen Raum mit kartesischem Koordinatensystem vorstellen, der an verschiedenen X-Y-Z Positionen unterschiedliche Arten von Annotationen gespeichert hat, die intraoperativ aufgenommen wurden.

Zu einem späteren Zeitpunkt kann der Chirurg dann über die Darstellungsvorrichtung 2 in einer Überlagerungsdarstellung U in einer Live-Darstellung der zeitaktuellen Aufnahme des Aufnahmesystems 12 das zuvor aufgenommene Annotationsobjekt zumindest positionskorrekt, etwa einen mittels eines Navigationszeigers festgelegten Punkt im Raum, oder auch lagekorrekt, etwa die 3D-Aufnahme einer Oberfläche, ausgeben. Eine chirurgische Navigation wird dadurch deutlich verbessert.

In der Fig. 2 ist eine kohärente Ansicht dargestellt, das bedeutet, dass zu einem ersten Zeitpunkt in einer ersten Lage des Aufnahmesystems ein Annotationsbefehl gegeben wurde, die Annotation entsprechend mit den zugehörigen Aufnahmedaten, nämlich wesentlichen Parametern der Kameraeinstellung wie dem Fokuspunkt und dem Zoom sowie der Roboterkonfiguration (Konfiguration der Glieder des Roboterarms 8 zueinander und zu der Roboterbasis 6) in der zentralen Annotationsdatenbank in dem globalen Koordinatensystem an der korrekten Position mit zusätzlicher Information zu der Orientierung gespeichert wurde und nach dem ersten Zeitpunkt der Roboter bewegt und ein Eingriff entsprechend weiter durchgeführt wurde.

Will nun der Chirurg zu der ersten Annotation zurückkehren, so wählt er mittels eines Ausgabebefehl, durch manuelle Eingabe bzw. Betätigung des Touch-Displays des OP-Monitors, die entsprechende Annotation aus und die Steuereinheit steuert auf Basis der hinterlegten Daten zu dem Annotationsobjekt den Roboterarm 8 in die dafür vorgesehene Konfiguration und damit in die selbe Lage des Aufnahmesystems 12 wie zu dem ersten Zeitpunkt, stellt die Kamera entsprechend der gespeicherten Kameraeinstellungen scharf und gibt eine Überlagerungsdarstellung mit einerseits dem zeitaktuellen 3D-Aufnahme 3DA und andererseits dem Teilausschnitt der gespeicherten 3D-Aufnahme 3DA aus, etwa eine Nebeneinanderdarstellung oder eine Überlagerung mittels Transparenz, um dem Chirurgen eine zeitliche Veränderung visuell zu präsentieren.

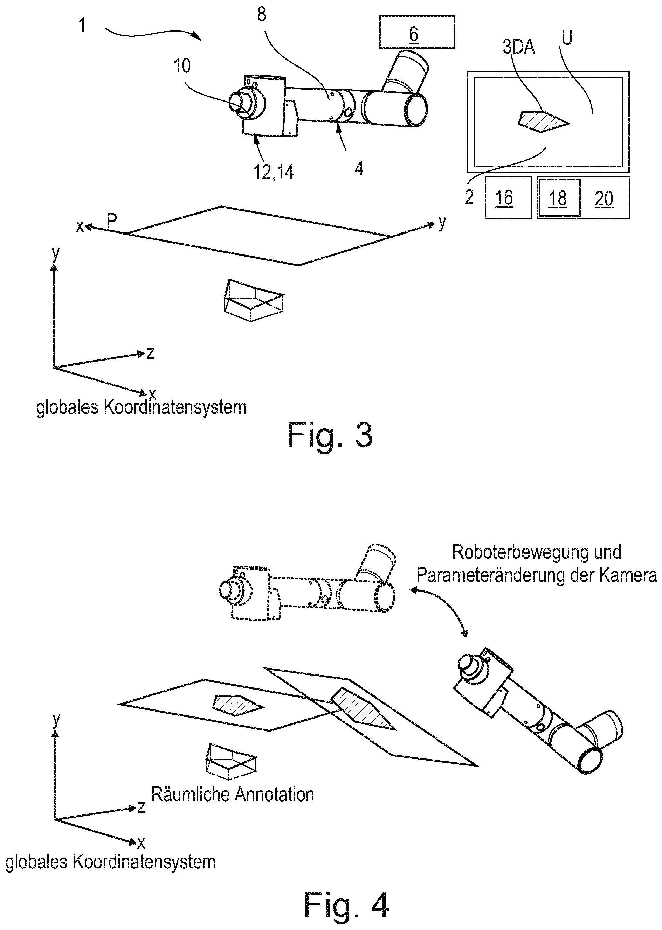

Fign. 3 und 4 zeigen in einer perspektivischen Ansicht ein chirurgisches Navigationssystem 1 einer weiteren bevorzugten Ausführungsform. Diese Ausführungsform unterscheidet sich lediglich dadurch, dass die Steuereinheit dafür angepasst ist, eine räumliche Annotation durchzuführen, wobei die Steuereinheit 20 dafür angepasst ist, auch aus einer zweiten Lage, die unterschiedlich zu einer ersten Lage ist (s. Fig. 4 erste Lage gestrichelt dargestellt, zweite Lage mit durchgezogenen Linien dargestellt) eine räumliche Darstellung, etwa durch entsprechende Matrizentransformationen, des zuvor aufgenommenen Annotationsobjekts darstellen zu können. Dabei hilft das zentrale globale Koordinatensystem mit hinterlegten Annotationsobjekten in Verbindung mit Bildanalyse und Aufnahmebearbeitungsverfahren die Annotation in korrekter Lage (lagekorrekt) in der zweiten Lage des Aufnahmesystems 12 einzublenden.

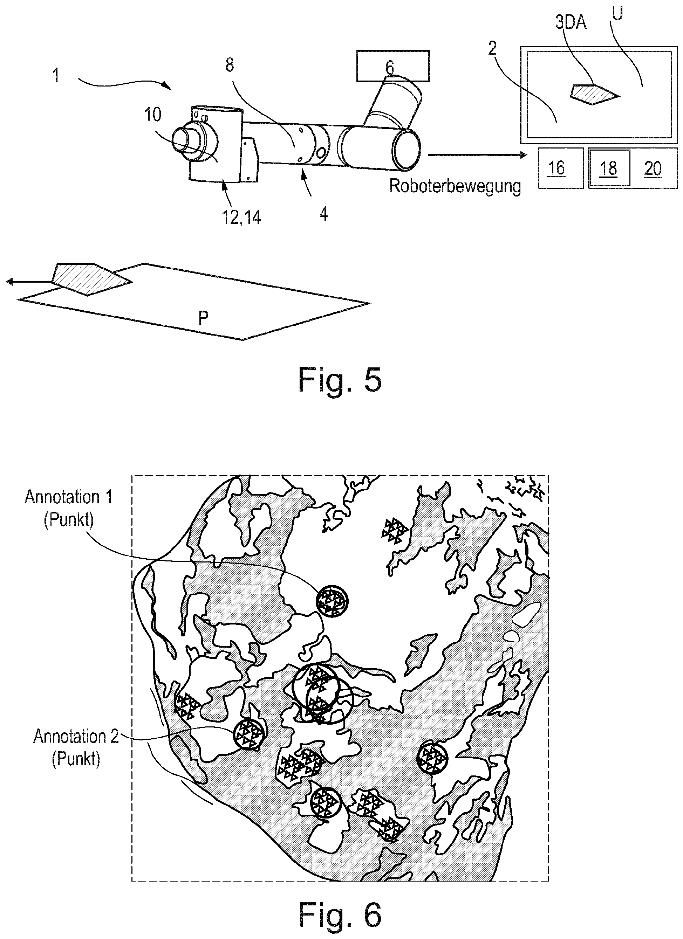

In Fig. 5 ist die Gegebenheit schematisch dargestellt, wenn der Roboter 4 bewegt wird und sich das zu annotierende Objekt aus dem Sichtbereich herausbewegt (etwa bei der kohärenten Ansicht).

In Fig. 6 ist schematisch ein Beispiel einer in der Annotationsdatenbank gespeicherten Annotation gezeigt. Ein Punkt im Raum des globalen Koordinatensystems mit Koordinaten X, Y und Z ist als Annotationsobjekt hinterlegt mit einer entsprechenden Beschriftung, die bedarfsgerecht in einer Überlagerungsdarstellung U mit eingeblendet werden kann.

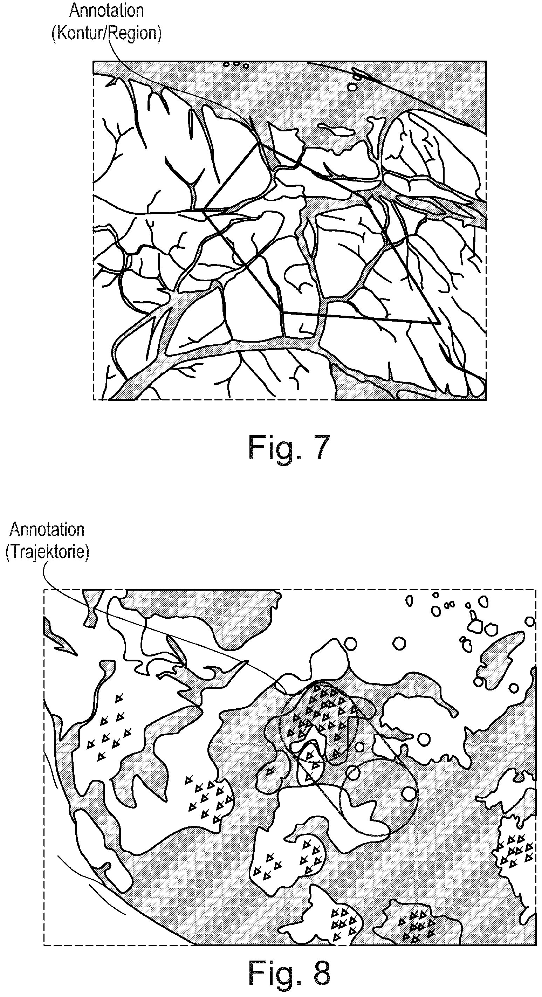

Fig. 7 zeigt eine weitere Alternative einer gespeicherten Annotation, in welcher ein Bereich einer Gewebeoberfläche als Fünfeck (farbig) markiert wurde. Bei einem Eingriff kann dem Chirurgen dann dieser Bereich mit eingeblendet werden.

Fig. 8 zeigt eine weitere Annotation in Form einer Trajektorie, welche in das Gewebe mit eingeblendet wird, um dem Chirurgen eine Führung bereitzustellen.

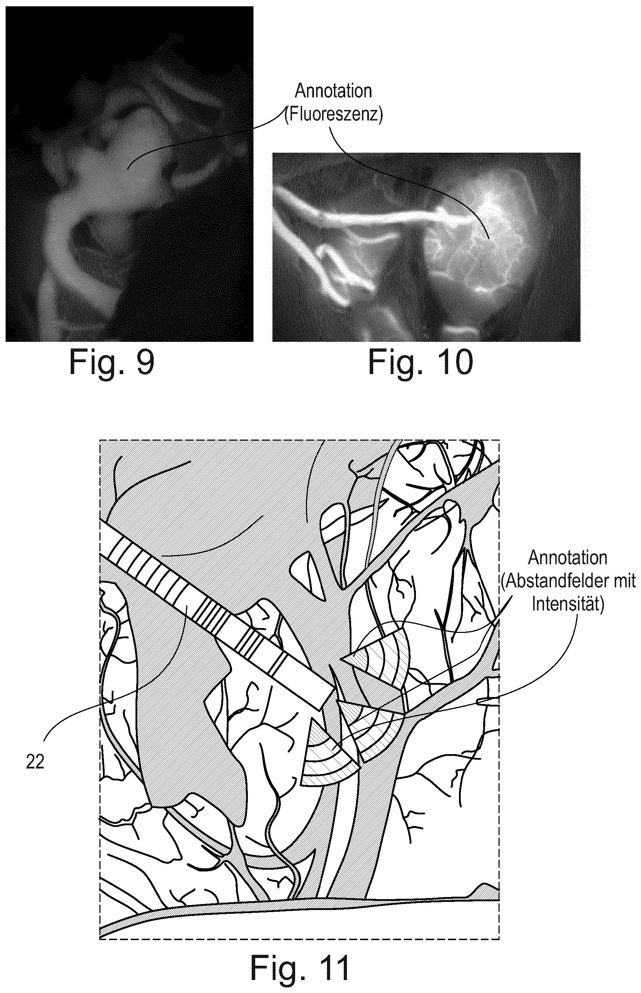

Fign. 9 und 10 zeigen 3D-Aufnahmen einer Gewebestruktur mit einer hinterlegten Fluoreszenz als Annotation.

In Fig. 11 ist ein Instrument 22, hier ein Pointer, mit einerseits Ringmarkierungen am Instrument 22 selbst, als auch Abstandsfeldern an der distalen Spitze des Instruments 22 als weitere Ausführungen von Annotationen gezeigt.

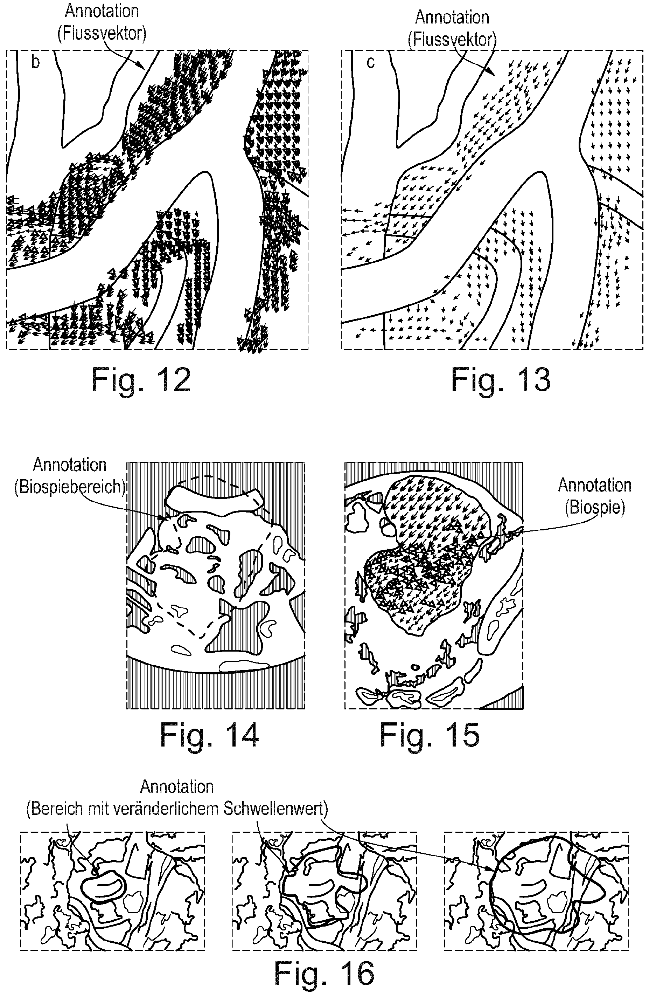

Fign 12 und 13 zeigen in einer hinterlegten 3D-Aufnahme 3DA Flussvektoren als Annotationen bzw. Annotationsobjekte.

Fig. 14 zeigt einen Bereich, in welchem eine Biopsie vorgenommen wurde und Fig. 15 wiederum zeigt in einer schematischen Ansicht ein Biopsie-Ergebnis mit unterschiedlichen Bereichen.

Fig. 16 zeigt (von links nach rechts) eine Änderung einer Umgrenzung/Grenze eines Bereichs, bei der ein Schwellenwert durch den Nutzer stetig erniedrigt wird. Mit anderen Worten kann der Nutzer einen Grenzwert vorgeben und anhand dieses Grenzwerts wird um ein Gewebebereich eine Grenze visualisiert. Dies stellt eine weitere Alternative einer Annotation dar.

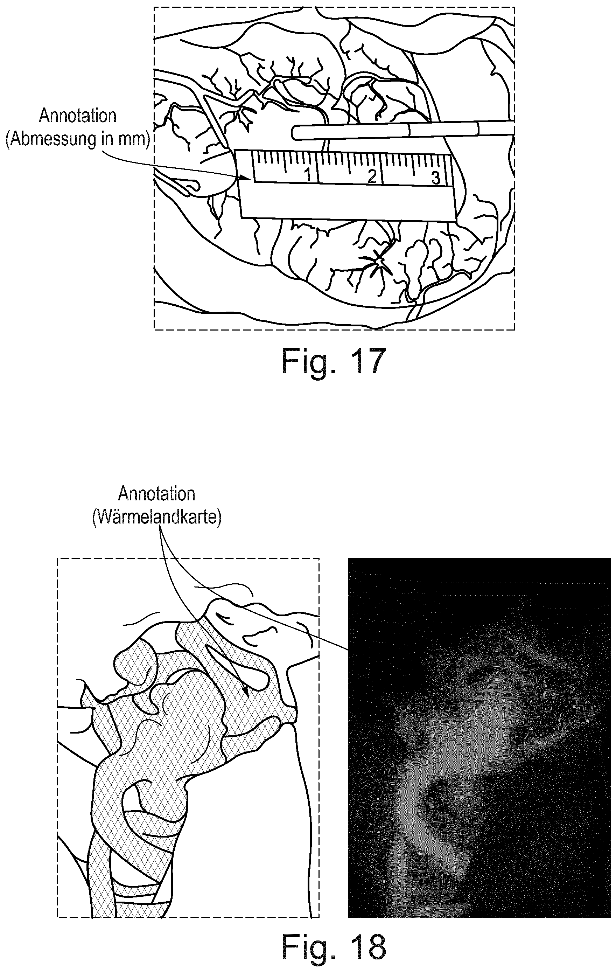

Fig. 17 zeigt eine Abmessung in mm als Annotation. Hierbei wurde ein steriles Messinstrument in Form eines Lineals innerhalb des Gewebes positioniert und eine 3D-Aufnahme durchgeführt. So kann zu einem späteren Zeitpunkt bei Bedarf, wenn das Aufnahmesystem wieder in einer ähnlichen Lage ist, die Abmessung eingeblendet werden, ohne dass erneut das Messinstrument real eingebunden werden muss.

Fig. 18 zeigt eine weitere Alternative einer Annotation in Form einer Wärmelandkarte.

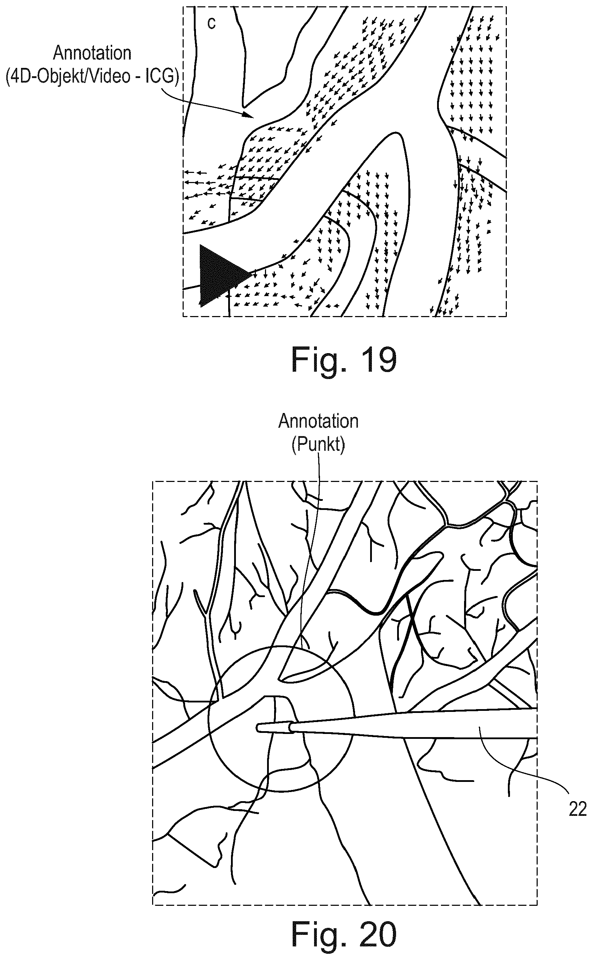

Fig. 19 zeigt ein Video eines Flusses als Annotation, so dass auch bewegte Bilder wiedergegeben werden können.

Fig. 20 zeigt ein Instrument 22 in der zeitaktuellen Aufnahme, während im Hintergrund des Instruments ein Markierungspunkt, hier durch einen Kreis dargestellt, als Annotation eingeblendet wird. Die Steuereinheit 20 ist dafür angepasst, Instrumente 22 zu erkennen und nach vorne in die Ebene zu setzen, so dass die Annotation das Instrument 22 nicht überdeckt.

### Bezugszeichenliste

- 1: Chirurgisches Navigationssystem
- 2: Darstellungsvorrichtung
- 4: Roboter
- 6: Roboterbasis
- 8: Roboterarm
- 10: Roboterkopf
- 12: Aufnahmesystem
- 14: Aufnahmeeinheit
- 16: Trackingsystem
- 18: Annotationssystem
- 20: Steuereinheit
- 22: Instrument

- P: Patient
- 3DA: 3D-Aufnahme
- U: Überlagerungsdarstellung

## Patentansprüche

1. Chirurgisches Navigationssystem (1) mit Annotationsfunktion für eine Eingabe von positionsbezogenen, insbesondere visuellen, medizinischen Annotationen oder Aufnahmen und/oder für eine Ausgabe von medizinischen Annotationen bei einem chirurgischen Eingriff bei einem Patienten (P), mit:
einer Darstellungsvorrichtung (2), insbesondere einem OP-Monitor, zur visuellen Ausgabe der medizinischen Annotationen;
zumindest einem Roboter (4) als Positioniersystem, mit einem an einer Roboterbasis (6) beweglich angebundenen, insbesondere mehrgliedrigem, Roboterarm (8) und einem an den Roboterarm (8) endständig angeordneten Roboterkopf (10),
ein an oder in dem Roboterkopf (10) angeordnetes stereoskopisches Aufnahmesystem (12), insbesondere einer Stereokamera, besonders bevorzugt einem Stereomikroskop, mit zumindest zwei zueinander beabstandeten Aufnahmeeinheiten (14), um eine 3D-Aufnahme (3DA) einer Struktur oder Oberfläche gegenüber dem Aufnahmesystem (12) durchzuführen und diese zusammen mit Aufnahmeparametern computerlesbar bereitzustellen;
ein Trackingsystem (16), das dafür angepasst ist, insbesondere mittelbar über die Lage des Roboterkopfs (10), die Lage des Aufnahmesystems (12) zu erfassen und nachzuverfolgen und die Lage des Aufnahmesystems (12) computerlesbar bereitzustellen;
ein Annotationssystem (18) mit einer Speichereinheit, in welcher eine zentrale digitale Annotationsdatenbank mit einem globalen Koordinatensystem für Annotationsobjekte gespeichert ist; und
eine Steuereinheit (20), die dafür angepasst ist, die bereitgestellte 3D-Aufnahme (3DA) zusammen mit den Aufnahmeparametern und der bereitgestellten Lage zu verarbeiten und basierend auf den bereitgestellten Aufnahmeparametern und der Lage des Aufnahmesystems (12) eine Lage der 3D-Aufnahme (3DA) in dem globalen Koordinatensystem zu bestimmen und über die Darstellungsvorrichtung (2) in einer Überlagerungsdarstellung (U) zumindest ein Annotationsobjekt der Annotationsdatenbank in der 3D-Aufnahme (3DA) zumindest positionskorrekt, insbesondere lagekorrekt, auszugeben und eine Navigation zu verbessern,
**dadurch gekennzeichnet, dass**
als Aufnahmeparameter eine Roboterarmkonfiguration des Roboters (4) und eine Kameraeinstellung des Aufnahmesystems (12) in der Annotationsdatenbank gespeichert werden, um den Roboter (4) zu einem späteren Zeitpunkt des Eingriffs in genau diese Position und Orientierung mit entsprechender Kameraeinstellung zu bewegen und in der Überlagerungsdarstellung (U) die zeitaktuelle 3D-Aufnahme sowie die zuvor gespeicherte 3D-Aufnahme als kohärente Referenz auszugeben, um eine zeitliche Veränderung zu visualisieren, und/oder
als Aufnahmeparameter ein Zoom(-faktor) und ein Brennpunkt als Kameraeinstellungen der Stereokamera des Aufnahmesystems (12) erfasst gespeichert werden, so dass über die durch das Trackingsystem nachverfolgte Lage des Aufnahmesystems (12) und über den Zoom sowie den Brennpunkt zumindest die Position eines Teilbereichs der 3D-Aufnahme präzise bestimmbar ist.

2. Chirurgisches Navigationssystem (1) nach Anspruch 1, **dadurch gekennzeichnet, dass** die Steuereinheit dafür angepasst ist, bei einem Annotationsbefehl, basierend auf den bereitgestellten Aufnahmeparametern und der Lage des Aufnahmesystems (12) eine Lage der 3D-Aufnahme (3DA) zu bestimmen, und als Annotationsobjekt zumindest einen Teilausschnitt der 3D-Aufnahme (3DA) zusammen mit den Aufnahmeparametern in der Annotationsdatenbank des Annotationssystems in dem globalen Koordinatensystem in der bestimmten Position, insbesondere Lage, zu speichern, um zu einem späteren Zeitpunkt über die Darstellungsvorrichtung (2) in einer Überlagerungsdarstellung (U) das Annotationsobjekt zumindest positionskorrekt, insbesondere lagekorrekt, auszugeben und eine Navigation zu verbessern.

3. Chirurgisches Navigationssystem (1) nach einem der vorstehenden Ansprüche, **dadurch gekennzeichnet, dass** die Steuereinheit (20) dafür angepasst ist, bei einem Ausgabebefehl zu einem in der Annotationsdatenbank gespeicherten Annotationsobjekt, das Aufnahmesystem (12) in die zu dem Annotationsobjekt gespeicherte Lage zu bewegen und in der Überlagerungsdarstellung (U) die zeitaktuelle 3D-Aufnahme (3DA) und das gespeicherte Annotationsobjekt, insbesondere den Teilausschnitt der gespeicherten 3D-Aufnahme (3DA), über die Darstellungsvorrichtung auszugeben.

4. Chirurgisches Navigationssystem (1) nach einem der vorstehenden Ansprüche, **dadurch gekennzeichnet, dass**
in der Speichereinheit ferner präoperative 3D-Aufnahmedaten, insbesondere CT-Aufnahmedaten und/oder MRT-Aufnahmedaten und/oder segmentierte Röntgenbilder, gespeichert sind;
das chirurgische Navigationssystem (1) dafür angepasst ist, den Patienten (P) gegenüber den präoperativen 3D-Aufnahmedaten zu registrieren; und
die Steuereinheit (20) dafür angepasst ist, in der Überlagerungsdarstellung (U) zu der 3D-Aufnahme (3DA) die präoperativen 3D-Aufnahmedaten für eine weitere Unterstützung mit einzublenden.

5. Chirurgisches Navigationssystem (1) nach einem der vorstehenden Ansprüche, **dadurch gekennzeichnet, dass** die Steuereinheit (20) dafür angepasst ist, bei einer Abweichung des in der Annotationsdatenbank gespeicherten Teilausschnitts der 3D-Aufnahme (3DA) gegenüber der zeitaktuellen 3D-Aufnahme (3DA), insbesondere mittels Bildanalyseverfahren, um eine vorgegebene Toleranz hinaus, eine Warnmeldung in der Überlagerungsdarstellung (U) mit einzublenden.

6. Chirurgisches Navigationssystem (1) nach einem der vorstehenden Ansprüche, **dadurch gekennzeichnet, dass** die Steuereinheit (20) dafür angepasst ist, ein Instrument (22) in der zeitaktuellen 3D-Aufnahme (3DA) zu erfassen und in der Überlagerungsdarstellung (U) in eine vordere Ebene zu setzen während das zumindest eine Annotationsobjekt in einer hinteren Ebene dargestellt wird, so dass in der Überlagerungsdarstellung (U) die Annotationsobjekte nicht mit dem Instrument (22) interferieren.

7. Chirurgisches Navigationssystem (1) nach einem der vorstehenden Ansprüche, **dadurch gekennzeichnet, dass** das Aufnahmesystem (12) eine zeitaktuelle 3D-Aufnahme (3DA) intraoperativ bereitstellt, welche einerseits über die Darstellungsvorrichtung ausgegeben wird und andererseits bei einem Annotationsbefehl in der Annotationsdatenbank gespeichert wird.

8. Chirurgisches Navigationssystem (1) nach einem der vorstehenden Ansprüche, **dadurch gekennzeichnet, dass** in der Annotationsdatenbank zu jedem Annotationsobjekt ein Parameter einer Transparenz zwischen 0 und 1 und damit zwischen einer Ausblendung und einer Einblendung hinterlegt ist, und die Steuereinheit (20) dafür angepasst ist, zusammen mit der zeitaktuellen 3D-Aufnahme (3DA) alle Annotationsobjekte entsprechend ihrer Transparenz in der Überlagerungsdarstellung zumindest positionskorrekt, insbesondere lagekorrekt, einzublenden.

9. Chirurgisches Navigationssystem (1) nach einem der vorstehenden Ansprüche, **dadurch gekennzeichnet, dass** die Steuereinheit (20) dafür angepasst ist, einen Schwellenwert zur Anzeige von Umrissen/Konturen als Annotation zu verändern, um einen Bereich für einen Nutzer bedarfsgerecht zu markieren.

10. Chirurgisches Navigationssystem (1) nach einem der vorstehenden Ansprüche, **dadurch gekennzeichnet, dass** das Aufnahmesystem (12) dafür angepasst ist, eine Kamerakalibrierung durchzuführen, um Informationen, insbesondere einer räumlichen Pyramide, der Steuereinheit (20) bereitzustellen, wie sich die gesammelten Annotationsobjekte in Bezug auf eine Änderung des Zooms oder des Brennpunkts bewegen, um die Steuereinheit (20) in die Lage zu versetzen, die Kameraeinstellungen während einer Bewegung zu ändern.

11. Chirurgisches Navigationssystem (1) nach einem der vorstehenden Ansprüche, **dadurch gekennzeichnet, dass** als assoziierte Daten des Annotationsobjekts eine inkrementelle ID, insbesondere eines nachverfolgten Zeigegerät und/oder eine Intensitätsinformation einer Fluoreszenzaufnahme und/oder eine konvexe Hülle von Trajektorien und/oder ein sphärisches Abstandsfeld in der Annotationsdatenbank gespeichert werden.

## Claims

1. A surgical navigation system (1) having an annotation function for inputting position-related, particularly visual, medical annotations or recordings, and/or for outputting medical annotations during a surgical procedure on a patient (P), comprising:
a display device (2), in particular a surgery monitor, for the visual output of the medical annotations;
at least one robot (4) as a positioning system, having a, particularly multi-link, robot arm (8) movably connected to a robot base (6) and a robot head (10) arranged at the end of the robot arm (8),
a stereoscopic recording system (12) arranged on or in the robot head (10), in particular a stereo camera, particularly preferred a stereo microscope, having at least two recording units (14) spaced apart from each other in order to perform a 3D recording (3DA) of a structure or surface relative to the recording system (12) and to provide the same together with recording parameters in a computer-readable form;
a tracking system (16) adapted, in particular indirectly via the location of the robot head (10), to detect and track the location of the recording system (12) and to provide the location of the recording system (12) in a computer-readable form;
an annotation system (18) having a storage unit in which a central digital annotation database with a global coordinate system for annotation objects is stored; and
a control unit (20) adapted to process the provided 3D recording (3DA) together with the recording parameters and the provided location and, based on the provided recording parameters and the location of the recording system (12), to determine a location of the 3D recording (3DA) in the global coordinate system and to output at least one annotation object of the annotation database in the 3D recording (3DA) at least in the correct position, particularly in the correct location, via the display device (2) in a superimposed display (U) and to improve navigation,
**characterized in that**
a robot arm configuration of the robot (4) and a camera setting of the recording system (12) are stored in the annotation database as recording parameters in order to move the robot (4) at a later point in time of the surgical procedure into exactly said position and orientation with a corresponding camera setting and to output the current 3D recording and the previously stored 3D recording as a coherent reference in the superimposed display (U) to visualize a change over time, and/or
a zoom (factor) and a focus detected as camera settings of the stereo camera of the recording system (12) are stored as recording parameters so that at least the position of a segment of the 3D recording can be precisely determined via the location of the recording system (12) tracked by the tracking system and via the zoom as well as the focus.

2. The surgical navigation system (1) according to claim 1, **characterized in that** the control unit is adapted, in the case of an annotation instruction, to determine a location of the 3D recording (3DA) based on the provided recording parameters and the location of the recording system (12), and to store, as an annotation object, at least a section of the 3D recording (3DA) together with the recording parameters in the annotation database of the annotation system in the global coordinate system in the specific position, in particular location, in order to output the annotation object at a later point in time at least in the correct position, in particular in the correct location, via the display device (2) in a superimposed display (U) and to improve navigation.

3. The surgical navigation system (1) according to one of the preceding claims, **characterized in that** the control unit (20) is adapted to move, in the case of an output instruction concerning an annotation object stored in the annotation database, the recording system (12) into the location stored relative to the annotation object and to output the current 3D recording (3DA) and the stored annotation object, in particular the section of the stored 3D recording (3DA), in the superimposed display (U) via the display device.

4. The surgical navigation system (1) according to one of the preceding claims, **characterized in that**
preoperative 3D recording data, in particular CT recording data and/or MRI recording data and/or segmented X-rays, are further stored in the storage unit;
the surgical navigation system (1) is adapted to register the patient (P) relative to the preoperative 3D recording data; and
the control unit (20) is adapted to display also the preoperative 3D recording data for further support in the superimposed display (U) relative to the 3D recording (3DA).

5. The surgical navigation system (1) according to one of the preceding claims, **characterized in that** the control unit (20) is adapted, when the section of the 3D recording (3DA) stored in the annotation database relative to the current 3D recording (3DA), in particular by means of an image analysis method, deviates beyond a preset tolerance, to display also a warning message in the superimposed display (U).

6. The surgical navigation system (1) according to one of the preceding claims, **characterized in that** the control unit (20) is adapted to detect an instrument (22) in the current 3D recording (3DA) and to put the same in a front plane in the superimposed position (U), while the at least one annotation object is shown in a rear plane, so that in the superimposed display (U), the annotation objects do not interfere with the instrument (22).

7. The surgical navigation system (1) according to one of the preceding claims, **characterized in that** the recording system (12) intraoperatively provides a current 3D recording (3DA) which, on the one hand, is output via the display device and in the case of an annotation instruction, on the other hand, is stored in the annotation database.

8. The surgical navigation system (1) according to one of the preceding claims, **characterized in that** a parameter of a transparency between 0 and 1 and, thus, between hiding and displaying is deposited for each annotation object in the annotation database, and the control unit (20) is adapted to display all annotation objects corresponding to their transparency together with the current 3D recording (3DA) at least in the correct position, in particular in the correct location, in the superimposed display.

9. The surgical navigation system (1) according to one of the preceding claims, **characterized in that** the control unit (20) is adapted to vary a threshold for indicating outlines/contours as an annotation in order to mark an area for a user as required.

10. The surgical navigation system (1) according to one of the preceding claims, **characterized in that** the recording system (12) is adapted to perform a camera calibration in order to provide the control unit (20) with information, in particular of a three-dimensional pyramid, about how the collected annotation objects move with respect to a change of the zoom or the focus in order to enable the control unit (20) to vary the camera settings during a movement.

11. The surgical navigation system (1) according to one of the preceding claims, **characterized in that** an incremental ID, in particular of a tracked pointing device, and/or an intensity information of a fluorescence recording, and/or a convex envelope of trajectories, and/or a spherical distance field are stored as associated data of the annotation object in the annotation database.

## Revendications

1. Système de navigation chirurgicale (1) avec fonction d'annotation pour une saisie d'annotations médicales liées à la position, en particulier visuelles, ou d'enregistrements, et/ou un affichage d'annotations médicales lors d'une intervention chirurgicale sur un patient (P), avec :
un dispositif de représentation (2), en particulier un moniteur OP, destiné à l'affichage visuel des annotations médicales ;
au moins un robot (4) en tant que de système de positionnement, avec un bras de robot (8) relié de manière mobile à une base de robot (6), en particulier à plusieurs segments, et une tête de robot (10) disposée à l'extrémité du bras de robot (8),
un système d'enregistrement stéréoscopique (12) disposé sur ou dans la tête de robot (10), en particulier une caméra stéréoscopique, le plus préférentiellement un stéréomicroscope, avec au moins deux unités d'enregistrement (14) espacées l'une de l'autre, pour réaliser un enregistrement 3D (3DA) d'une structure ou d'une surface par rapport au système d'enregistrement (12) et mettre celui-ci à disposition sous une forme lisible par ordinateur avec les paramètres d'enregistrement ;
un système de suivi (16) qui est adapté pour détecter et suivre la position du système d'enregistrement (12), en particulier indirectement par l'intermédiaire de la position de la tête de robot (10), et fournir la position du système d'enregistrement (12) sous une forme lisible par ordinateur ;
un système d'annotation (18) avec une unité de stockage dans laquelle une base de données d'annotation numérique centrale est stockée avec un système de coordonnées global pour objets d'annotation ; et
un dispositif de commande (20) qui est adapté pour traiter l'enregistrement 3D (3DA) mis à disposition avec les paramètres d'enregistrement et la position fournie, et déterminer, sur la base des paramètres d'enregistrement fournis et de la position du système d'enregistrement (12), une position de l'enregistrement 3D (3DA) dans le système de coordonnées global, et afficher, par l'intermédiaire du dispositif de visualisation (2), dans une représentation superposée (U), au moins un objet d'annotation de la base de données d'annotations dans l'enregistrement 3D (3DA) avec une position au moins correcte, en particulier une orientation correcte, et améliorer une navigation,
**caractérisé en ce que**
une configuration de bras de robot du robot (4) et un réglage de caméra du système d'enregistrement (12) sont enregistrés dans la base de données d'annotation pour déplacer le robot (4), à un moment ultérieur de l'intervention, exactement dans cette position et cette orientation avec le réglage de caméra correspondant, et afficher dans la représentation superposée (U) l'enregistrement 3D actuel ainsi que l'enregistrement 3D précédemment enregistré en tant que référence cohérente, pour visualiser un changement dans le temps, et/ou
un (facteur de) zoom et une distance focale sont enregistrés en tant que paramètres d'enregistrement et réglages de caméra stéréoscopique du système d'enregistrement (12), de sorte qu'au moins la position d'une partie de l'enregistrement 3D peut être déterminée avec précision à partir de la position du système d'enregistrement (12) suivie par le système de suivi, ainsi que du zoom et de la distance focale.

2. Système de navigation chirurgicale (1) selon la revendication 1, **caractérisé en ce que** le dispositif de commande est adapté pour, lors d'une commande d'annotation, déterminer une position de l'enregistrement 3D (3DA) sur la base des paramètres d'enregistrement fournis et de la position du système d'enregistrement (12), et pour enregistrer, en tant qu'objet d'annotation, au moins une partie de l'enregistrement 3D (3DA) avec les paramètres d'enregistrement dans la base de données d'annotation du système d'annotation, dans le système de coordonnées global, à la position déterminée, en particulier à l'emplacement déterminé, pour pouvoir, par l'intermédiaire du dispositif d'affichage (2), afficher ultérieurement l'objet d'annotation dans une représentation superposée (U) avec une position au moins correcte, en particulier un emplacement correct, et améliorer la navigation.

3. Système de navigation chirurgicale (1) selon l'une quelconque des revendications précédentes, **caractérisé en ce que** le dispositif de commande (20) est adapté pour, lors d'une commande d'affichage d'un objet d'annotation enregistré dans la base de données d'annotation, déplacer le système d'enregistrement (12) vers la position enregistrée pour l'objet d'annotation et afficher, dans la représentation superposée (U), l'enregistrement 3D (3DA) actuel et l'objet d'annotation enregistré, en particulier la partie de l'enregistrement 3D (3DA) enregistrée, par l'intermédiaire du dispositif de représentation.

4. Système de navigation chirurgicale (1) selon l'une quelconque des revendications précédentes, **caractérisé en ce que**
des données d'enregistrement 3D préopératoires (1), en particulier des données d'enregistrement par tomodensitométrie et/ou des données d'enregistrement par résonance magnétique et/ou des radiographies segmentées sont en outre stockées dans l'unité de stockage ;
le système de navigation chirurgicale (1) est adapté pour enregistrer le patient (P) par rapport aux données d'enregistrement 3D préopératoires ; et
le dispositif de commande (20) est adapté pour afficher, dans la représentation superposée (U) de l'enregistrement 3D (3DA), les données d'enregistrement 3D préopératoires pour une assistance supplémentaire.

5. Système de navigation chirurgicale (1) selon l'une quelconque des revendications précédentes, **caractérisé en ce que** le dispositif de commande (20) est adapté pour, en cas d'écart de la partie de l'enregistrement 3D (3DA) enregistrée dans la base de données d'annotations par rapport à l'enregistrement 3D (3DA) actuel, en particulier au moyen d'un procédé d'analyse d'image, au-delà d'une tolérance prédéfinie, afficher un message d'avertissement dans la représentation superposée (U).

6. Système de navigation chirurgicale (1) selon l'une quelconque des revendications précédentes, **caractérisé en ce que** le dispositif de commande (20) est adapté pour détecter un instrument (22) dans l'enregistrement 3D actuel (3DA) et le placer dans un plan avant de la représentation superposée (U) tandis qu'au moins un objet d'annotation est représenté dans un plan arrière, de sorte que, dans la représentation superposée (U), les objets d'annotation n'interfèrent pas avec l'instrument (22).

7. Système de navigation chirurgicale (1) selon l'une quelconque des revendications précédentes, **caractérisé en ce que** le système d'enregistrement (12) fournit en temps réel un enregistrement 3D (3DA) en peropératoire, qui est d'une part affichée par l'intermédiaire du dispositif de représentation et d'autre part enregistrée dans la base de données d'annotations lors d'une commande d'annotation.

8. Système de navigation chirurgicale (1) selon l'une des revendications précédentes, **caractérisé en ce que,** dans la base de données d'annotation, un paramètre de transparence compris entre 0 et 1, et donc entre une opacité totale et une transparence totale, est enregistré pour chaque objet d'annotation, et le dispositif de commande (20) est adapté pour afficher, conjointement avec l'enregistrement 3D (3DA) actuel, tous les objets d'annotation dans la représentation superposée, au moins avec une position correcte, en particulier une orientation correcte, en fonction de leur transparence.

9. Système de navigation chirurgicale (1) selon l'une quelconque des revendications précédentes, **caractérisé en ce que** le dispositif de commande (20) est adapté pour modifier une valeur seuil d'affichage des contours en tant qu'annotation, pour marquer une zone pour un utilisateur selon le besoin.

10. Système de navigation chirurgicale (1) selon l'une quelconque des revendications précédentes, **caractérisé en ce que** le système d'enregistrement (12) est adapté pour effectuer un étalonnage de caméra pour fournir des informations, en particulier une pyramide spatiale, à l'unité de commande (20) sur la manière dont les objets d'annotation collectés se déplacent par rapport à une modification du zoom ou du point focal, pour permettre à le dispositif de commande (20) de modifier les réglages de caméra pendant un mouvement.

11. Système de navigation chirurgicale (1) selon l'une quelconque des revendications précédentes, **caractérisé en ce qu'**un ID incrémental, en particulier d'un dispositif de pointage suivi et/ou une information d'intensité d'une image fluorescente et/ou une enveloppe convexe de trajectoires et/ou un champ de distance sphérique sont enregistrés dans la base de données d'annotation en tant que données associées à l'objet d'annotation.
